(19) Europäisches Patentamt
European Patent Office
Office européen des brevets

(11) Veröffentlichungsnummer: **0 124 067**
**B1**

(12) # EUROPÄISCHE PATENTSCHRIFT

(45) Veröffentlichungstag der Patentschrift: **17.12.86**

(21) Anmeldenummer: **84104595.8**

(22) Anmeldetag: **24.04.84**

(51) Int. Cl.⁴: **C 07 D 207/335**, C 07 D 401/06, C 07 D 401/12, C 07 D 403/06, C 07 D 403/12, C 07 D 403/14, A 61 K 31/40

(54) **2-(Aminoalkyl)-pyrrol-Derivate, Verfahren zu ihrer Herstellung und ihre Verwendung.**

(30) Priorität: **04.05.83 DE 3316187**
**03.02.84 DE 3403731**

(43) Veröffentlichungstag der Anmeldung: **07.11.84 Patentblatt 84/45**

(45) Bekanntmachung des Hinweises auf die Patenterteilung: **17.12.86 Patentblatt 86/51**

(84) Benannte Vertragsstaaten: **AT BE CH DE FR GB IT LI NL SE**

(56) Entgegenhaltungen:
**DE-A-2 062 915**
**DE-A-2 419 535**
**DE-A-2 645 171**

**LIEBIGS ANNALEN DER CHEMIE, 1980, VERLAG CHEMIE, Weinheim. H. STETTER et al.: "Herstellung bi-und tricyclischer Pyrrol-Systeme" Seiten 703-714**
**JOURNAL OF MEDICINAL CHEMISTRY, Band 21, No. 1, 1, Jänner 1978. T. HARA et al.: "Diazepines. 5. Synthesis and Biological Action of 6-Phenyl-4H-pyrrolo (1,2-a) (1,4) benzodiazepines", Seiten 263-268**
**JOURNAL OF HETETOCYCLIC CHEMISTRY, Band 14, No. 1, Februar 1977. Y. KAYAMA et al.: "Diazepines II. A New Synthesis of 4H-Pyrrolo (1,2a)(1,4) benzodiazepine" Seiten 171-172**

(73) Patentinhaber: **CASSELLA Aktiengesellschaft, Hanauer Landstrasse 526, D-6000 Frankfurt am Main 61 (DE)**

(72) Erfinder: **Zoller, Gerhard, Dr., Langwiese 16, D-6457 Maintal (DE)**
Erfinder: **Beyerle, Rudi, Dr., An der Pfaffenmauer 44, D-6000 Frankfurt/Main 60 (DE)**
Erfinder: **Schindler, Ursula, Dr., Reinhardswaldweg 1, D-6082 Mörfelden- Waldorf 2 (DE)**
Erfinder: **Nitz, Rolf- Eberhard, Dr., Heinrich-Bingemer- Weg 64, D-6000 Frankfurt/Main 60 (DE)**
Erfinder: **Martorana, Piero A., Dr., Kaiser-Friedrich- Promenade 108, D-6380 Bad Homburg (DE)**

(74) Vertreter: **Urbach, Hans- Georg, Dr., Hanauer Landstrasse 526, D-6000 Frankfurt am Main 61 (DE)**

(56) Entgegenhaltungen: (Fortsetzung)
**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 124 067 B1

LIBER, STOCKHOLM 1986

**Beschreibung**

Die Erfindung betrifft neue 2-(Aminoalkyl)-pyrrol-derivate der allgemeinen Formel

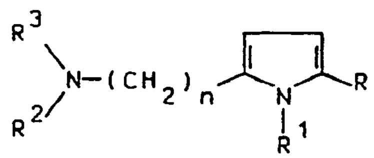

(I)

worin

R: Wasserstoff, Alkyl($C_1$-$C_5$), Phenyl;

$R^1$: Wasserstoff, Alkyl($C_1$-$C_5$), Cyano-alkyl($C_1$-$C_4$), Alk-oxy($C_1$-$C_4$)-carbonyl, Alkoxy($C_1$-$C_4$)-carbonyl-alkyl($C_1$-$C_4$), $R^7$($R^8$)N-carbonyl-alkyl($C_1$-$C_4$), Phenyl, Phenyl-alkyl($C_1$-$C_4$), wobei das Phenyl oder das Phenyl des Phenylalkyls auch durch Halogen, Alkoxy($C_1$-$C_4$), Alkyl($C_1$-$C_4$), $R^4$($R^5$)N-, Hydroxy, Merkapto, Alkylmerkapto($C_1$-$C_4$), Nitro, Cyano, Alkoxy($C_1$-$C_4$)-carbonyl, Alkoxy($C_1$-$C_4$)-carbonyl-alkyl($C_1$-$C_4$) ein- oder mehrfach substituiert sein kann, Imidazolyl-alkyl($C_1$-$C_4$), $R^4$($R^5$)N-$R^6$-, Acetyl-amino-ethyl, Hydroxy-carbonyl-alkyl($C_1$-$C_4$), 1-(Alkoxy($C_1$-$C_4$)-carbonyl)-2-mercapto-ethyl, 1-(Alkoxy($C_1$-$C_4$)-carbonyl)-2-hydroxy-ethyl, 1-(Alkoxy($C_1$-$C_4$)-carbonyl)-2-alkoxy-($C_1$-$C_4$)-ethyl, 1-(Alkoxy($C_1$-$C_4$)-carbonyl)-2-alkylthio-($C_1$-$C_4$)-ethyl, 1-(Alkoxy($C_1$-$C_4$)-carbonyl)-2-(alkyl($C_1$-$C_4$)-carbonyl-thio)-ethyl, 1-(Alkoxy($C_1$-$C_4$)carbonyl)-2-(alkyl($C_1$-$C_4$)-carbonyl-oxy)-ethyl, 1-(Alkoxy($C_1$-$C_4$)-carbonyl)-2-imidazolyl-ethyl, 1-(Alkoxy($C_1$-$C_4$)-carbonyl)-2-indolyl-ethyl, 1-(Alkoxy($C_1$-$C_4$)-carbonyl)-2-phenyl-ethyl;

$R^2$, $R^3$: unabhängig voneinander Wasserstoff, Alkyl($C_1$-$C_5$), Alkanoyl($C_1$-$C_5$); durch Amino, Alkoxy($C_1$-$C_4$), Hydroxy, Alkanoyloxy($C_1$-$C_4$), Phenyl, Halogen, ein-, zwei- oder dreifach substituiertes Alkanoyl($C_2$-$C_5$), Benzoyl, durch Halogen, Alkoxy($C_1$-$C_4$), Alkyl($C_1$-$C_4$), Amino, $R^4$($R^5$)N-, Hydroxy, Alkanoyloxy($C_1$-$C_4$), Merkapto, Alkylmercapto ($C_1$-$C_4$), Nitro, Cyano, Alkoxy($C_1$-$C_4$)-carbonyl, Amidosulfonyl ein- oder mehrfach substituiertes Benzoyl, Pyridylcarbonyl, Cycloalkylcarbonyl mit 5 bis 7 C-Atomen im Cycloalkylrest, wobei eine $-CH_2$-Gruppe auch durch -O-, -S- oder -N($R^{10}$)- ersetzt sein kann; (4-Chlorphenoxy)-acetyl; oder $R^2$ und $R^3$ zusammen vicinales Phenylendicarbonyl, Phenylen-1-sulfonyl-2-carbonyl, Alkylen($C_2$-$C_8$)-di-carbonyl, Alkenylen($C_4$-$C_8$)-dicarbonyl, wobei die Kohlenstoffkette des Alkylen- und Alkenylen-dicarbonyls auch durch -O-, -S-, -N($R^4$)- ein- oder mehrfach unterbrochen oder ein Spiroatom enthalten kann, über das ein 4-, 5-, 6- oder 7-Ring angebunden sein kann, Phenylen-1-sulfi-nyl-2-carbonyl, Phenylen-1-sulfenyl-2-carbonyl;

$R^4$,$R^5$: Wasserstoff, Alkyl($C_1$-$C_4$);

$R^6$: Alkylen($C_1$-$C_4$);

$R^7$, $R^8$: unabhängig voneinander Wasserstoff, Alkyl($C_1$-$C_4$) oder zusammen mit dem Stickstoffatom, an das sie gebunden sind, einen 5- oder 6-gliedrigen, gesättigten heterocyclischen Ring bilden, der auch noch -O- oder

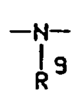

enthalten kann;

$R^9$: Wasserstoff, Alkyl($C_1$-$C_4$), Alkoxy($C_1$-$C_4$), Phenyl, Al-kyl($C_1$-$C_4$)-phenyl, Alkoxy($C_1$-$C_4$)-phenyl;

$R^{10}$: Wasserstoff, Alkanoyl($C_1$-$C_5$);

n: 1, 2 oder 3;

bedeuten, sowie ihre Säureadditionssalze, wobei für die Fälle, in denen $R^2$ und $R^3$ zusammen 1,2-Phenylen-dicarbonyl und gleichzeitig R Methyl oder Ethyl und n die Zahl 1 bedeuten, $R^1$ kein Ethoxy-carbonylmethyl, Ethoxycarbonyl oder o-Methoxy-carbonylphenyl sein kann und wobei für die Fälle, in denen $R^2$ und $R^3$ zusammen 1,2-Phenylen-dicarbonyl und gleichzeitig R Methyl oder Ethyl und n die Zahl 2 bedeuten, $R^1$ kein Ethoxy-carbonyl oder Ethoxy-carbonylmethyl sein kann und wobei für die Fälle, in denen $R^2$ und $R^3$ zusammen 1,2-Phenylen-dicarbonyl und gleichzeitig n die Zahl 1 und R Phenyl bedeuten, $R^1$ kein O-Methoxy-carbonylphenyl oder Ethoxy-carbonylmethyl sein kann.

Die Erfindung betrifft auch Verfahren zur Herstellung der Verbindungen I und ihrer Säureadditionssalze sowie ihre Verwendung als Arzneimittel;

Die Alkyl-, Alkoxy-, Alkanoyl-, Alkanoyloxy-, Alkylen-, Alkenylen-, Alkylmerkapto-, Alkoxycarbonylreste können, auch wenn sie in Verbindung mit anderen Resten auftreten, geradkettig oder verzweigt sein. Für Phenylalkyl kommt vor allem Phenethyl, vorzugsweise Benzyl, in Betracht;

Halogen ist vor allem Fluor, Chlor oder Brom. Unter vicinalem Phenylen-dicarbonyl werden solche Phenylendicarbonyl-reste verstanden, die sich von aromatischen 1,2-Dicarbonsäuren ableiten, z.B; Phthaloyl. Alkylendicarbonyl ist insbesondere Alkylen-1,2-, -1,3-oder -1,9-dicarbonyl, vorzugsweise Succinyl; Alkenylendicarbonyl ist insbesondere Alkenylen-1,2-, -1,3- oder -1,9-dicarbo-nyl, z.B. Citraconoyl, vorzugsweise aber Maleoyl.

Beispiele für die für R stehenden Alkylreste sind Methyl, Ethyl, Isopropyl. Für den Substituenten R ist Methyl bevorzugt; Beispiele für Substituenten, die für $R^1$ stehen können, sind: Wasserstoff, Methyl, Ethyl, Propyl, Isopropyl, Butyl(l), Butyl(2), Isobutyl, tert.Butyl, Isopentyl, Aminomethyl, 2-Aminoethyl, 3-Amino-n-propyl, 2-(N,N-Dimethylamino)-ethyl, 2-(N,N-Diethylamino)-ethyl, Cyanomethyl, 2°Cyanoethyl, 3°Cyano-propyl, Methoxy-carbonyl, n-Propoxy-carbonyl, i-Propoxy-carbonyl, n-Butoxy-carbonyl, t-Butoxy-carbonyl, Methoxycarbonylmethyl, n-Propoxycarbonylmethyl, i-Butoxycarbonylmethyl, 2-(Methoxycar-bonyl)-ethyl, 2-(Ethoxycarbonyl)-ethyl, 2-(i-Propoxycar-bonyl)-ethyl, 2-(Methoxycarbonyl)-propyl, 2-(Ethoxycar-bonyl)-propyl, Aminocarbonylmethyl, 2-(Aminocarbonyl)-ethyl, N,N-Dimethylaminocarbonylmethyl, (N,N-Dimethylaminoethyl)-aminocarbonyl-methyl, Ethylaminocarbonyl ethyl, Morpholinocarbonylmethyl, 4-Methylpiperazin(1)yl-carbonylmethyl, 4-Phenylpiperazin(1)yl-carbonyl-methyl, 4-(o-, m-, p-Methoxyphenyl)-piperazin(1)yl-carbonylmethyl, Pyrrolidino-carbonyl-methyl, N,N-Dimethylaminocarbonyl-ethyl, 2-(4-Methylpiperazin(1)yl-carbonyl)-ethyl, Phenyl, 2-Methylphenyl, 3-Methylphenyl, 4-Methylphenyl, 2-Methoxyphenyl, 3-Methoxyphenyl, 4-Methoxyphenyl, 3,4-Dimethoxyphenyl, o-, m-, p-Chlorphenyl, 4-Dimethylaminophenyl, 4-Mercaptophenyl, 4-Methoxybenzyl, 1-(Methoxy-carbonyl)-2-mercapto-ethyl, 1-(Ethoxy-carbonyl)-2-phenyl-ethyl, 1-(i-Butoxy-carbonyl)-2-(3-indolyl)-ethyl, 1-(Propoxy-carbonyl)-2-hydroxy-ethyl, 1-(Methoxy-carbo-nyl)-2-hydroxy-ethyl, 1-(Methoxy-carbonyl)-2-methoxy-ethyl, 1-(Methoxy-carbonyl)-2-butoxyethyl, 1-(i-Butoxy-carbonyl)-2-ethoxy-ethyl, 1-(Ethoxycarbonyl)-2-(methyl-thio)-ethyl, 1-(Propoxy-carbonyl)-2(propylthio)-ethyl, 1-(Ethoxy-carbonyl)-2-(acetylthio)ethyl, 1-(Methoxycar-bonyl)-2-(formyl-thio)ethyl, 1-(Ethoxy-carbonyl)-2(acet-oxy)-ethyl, 1-(Methoxy-carbonyl)-2-(formyloxy)ethyl, 1-(Ethoxycarbonyl)-2-(4(5)imidazolyl)-ethyl, 1-(Propoxy-carbonyl)-2-(3-indolyl)-ethyl, 1-Methoxy-carbonyl)-2-phenyl-ethyl.

Bevorzugte Reste für den Substituenten $R^1$ sind: Wasserstoff, $R^4R^5N$-$R^6$-, Benzyl, Phenethyl, wobei die Phenylgruppe in dem Benzyl- oder Phenethylrest auch durch ein, zwei oder drei Methyl- und/oder Methoxygruppen substituiert sein kann, Phenyl, das auch durch Alkoxy($C_1$-$C_4$)-carbonyl, Alkyl($C_1$-$C_4$), Halogen, Alkoxy($C_1$-$C_4$), Hydroxy mono-, di- oder trisubstituiert sein kann, Methoxy-carbonyl-phenyl, Methoxy-carbonyldimethoxyphenyl, Methoxycarbonyl-trimethoxyphenyl, Imidazolylethyl, Cyanoethyl, Methoxycarbonyl-methyl, Acetaminoethyl. Beispiele für bevorzugte Reste $R^1$ sind: Wasserstoff, Dimethoxyphenylethyl, insbesondere 3,4-Dimethoxy-phenyl-ethyl, Methoxycarbonyl-methyl, Imidazolylethyl, insbesondere 2-(4-Imi-dazolyl)-ethyl, 3-(1-Imidazolyl)-propyl, Acetaminoethyl, (Methoxy-carbonyl)dimethoxy-phenyl, insbesondere 2-(Meth-oxycarbonyl)-4,5-dimethoxy-phenyl, 2-(Dimethylamino)-ethyl, Aminocarbonylmethyl, 2-Cyanethyl, 2-Methoxy-carbonyl-phenyl, 2-Methoxy-carbonyl-4,5,6-trimethoxy-phenyl, 2-Methoxy-carbonyl-4,5-dimethoxyphenyl.

In den für $R^1$ stehenden Resten 1-(Alkoxy($C_1$-$C_4$)-carbonyl)-2-imidazolyl-ethyl und 1-(Alkoxy($C_1$-$C_4$)-carbonyl)-2-indolyl-ethyl sind die heterocyclischen Reste insbesondere 4(5)-Imidazolyl- und 3-Indolyl-Reste;

Für $R^2$ und $R^3$ können beispielsweise auch unabhängig voneinander stehen: Wasserstoff, Methyl, Ethyl, Propyl, Isopropyl, Butyl(1), Butyl(2), Isobutyl, Isopentyl, Pentyl, Formyl, Acetyl, Propionyl, Butyryl, Isobutyryl, Valeryl, Isovaleryl, Pivaloyl, Chloracetyl, Aminoacetyl, Methoxyacetyl, Dichloracetyl, Trichloracetyl, 3-Methoxypropylcarbonyl, Benzoyl, o-, m-, p-Methylbenzoyl, Dimethylbenzoyl, o-, m-, p-Methoxybenzoyl, Dimethoxybenzoyl, 3,4-Dimethoxybenzoyl, o-, m-, p-Chlorbenzoyl, o-, m-, p-Brombenzoyl, o-, m-, p-Aminobenzoyl, o-, m-, p-Hydroxybenzoyl, 4-Hydroxy-3-methoxy-benzoyl, 4-Acetoxy-3-methoxy-benzoyl, 2-Phenyl-2-hydroxyacetyl (Mandeloyl), 2-Phenyl-2-acetoxy-acetyl, 2-Phenyl-3-hydroxy-propionyl (Tropoyl), 2-Phenyl-3-acetoxy-propionyl, 2-, 3-, 4-Pyridyl-carbonyl.

Weitere Beispiele für $R^2$ und/oder $R^3$ sind: Cyclopentyl-carbonyl, Cyclohexyl-carbonyl, Cycloheptyl-carbonyl, Pyrrolidin-2-yl-carbonyl, 1-Formyl-pyrrolidin-2-yl-carbonyl, 1-Acetyl-pyrrolidin-2-yl-carbonyl, Pyrrolidin-3-yl-carbonyl, Piperidin-2-yl-carbonyl, Piperidin-3-yl-carbonyl, Piperidin-4-yl-carbonyl, 4-Chlorphenoxy-acetyl, 2-, 3- oder 4-Nitrobenzoyl, 2-, 3- oder 4-Cyanobenzoyl, 2-, 3-oder 4-Alkoxy($C_1$-$C_4$)-carbonyl-benzoyl, wie z.B. 2-, 3- oder 4-Methoxy-carbonyl-benzoyl, 2-, 3- oder 4-Ethoxy-carbonyl-benzoyl, 2-, 3- oder 4-Propoxy-carbonyl-benzoyl, 2-, 3- oder 4-sec-Butoxy-carbonyl-benzoyl.

Beispiele für $R^2$ und $R^3$ zusammen sind: Phthaloyl-dicarbonyl, Pyridyl-2,3-dicarbonyl, Succinyl, Glutaryl, Maleoyl, Citraconoyl, Buten-1,4-dicarbonyl, Phenyl-1-sulfo-nyl-2-carbonyl, 2-Thiabutandioyl (= $-CO-S-CH_2-CO-$), 2,2-Tetramethylen-propan-1,3-dicarbonyl. Bevorzugte Reste für $R^2$/$R^3$ sind z.B.: H/H, Nicotinoyl/H, 3,4-Dimethoxybenzoyl/H, Acetamino/H, Phenylen-1-sulfonyl-2-carbonyl, Phthaloyl, Phenylen-1-sulfenyl-2-carbonyl, Phenylen-1-sulfinyl-2-carbonyl.

Der für $R^2$ und/oder $R^3$ stehende Cycloalkylcarbonylrest besitzt vorzugsweise, auch wenn eine $-CH_2$-Gruppe durch -O-, -S- oder -N($R^{10}$)- ersetzt ist, 5 C-Atome im Cycloalkylrest;

Von den Verbindungen der Formel I sind solche bevorzugt, bei denen R ein Methylrest und/oder bei denen R Wasserstoff bedeutet oder zusammen mit dem Rest $R^3$ einen Rest darstellt;

Die 2-(Aminoalkyl)-pyrrol-derivate der Formel I werden in an sich bekannter Weise dadurch hergestellt, daß ein 1,4-Diketon der Formel II mit einem Amin der Formel III

$$(R^2)(R^3)N-(CH_2)_n-CO-CH_2CH_2-CO-R + H_2NR^1 \longrightarrow (I)$$
$$(II) \qquad\qquad (III)$$

in einem geeigneten inerten Lösungsmittel oder Lösungsmittelgemisch bei Temperaturen von 0 bis 200°C umgesetzt wird. In den Verbindungen II und III haben die Reste R, $R^1$, $R^2$ und $R^3$ sowie n die bereits genannten Bedeutungen.

In vielen Fällen sind Temperaturen von 20 bis 100°C ausreichend. Geeignete Lösungsmittel sind z.B. Alkohole, insbesondere solche mit 1 bis 6 C-Atomen, wie z.B. Methanol, Ethanol, i- und n-Propanol, i-, sec- und tert-Butanol, n-, i-, sec-, tert-Pentanol, n-Hexanol, Cyclopentanol, Cyclohexanol; Ether, insbesondere solche mit 2 bis 8 C-Atomen im Molekül, wie z.B. Diethylether, Methylethylether, Di-n-propylether, Diisopropylether, Methyl-n-butylether, Ethylpropylether, Dibutylether, Tetrahydrofuran, 1,4-Dioxan, 1,2-Dimethoxyethan, Bis-β-methoxyethylether; Polyether, wie z.B. Polyethylenglykole mit einem Molekulargewicht bis ca. 600; Oligoethylen-glykol-dimethylether, wie z.B. Pentaglyme; aliphatische Carbonsäuren, insbesondere Ameisen- und Essigsäure; Glykole und teilweise veretherte Glykole, wie z.B. Ethylenglykol, Propylenglykol, Trimethylenglykol, Ethylenglykol-monomethylether, Ethylenglykol-monoethylether, Diethylenglykolmonoethyl-ether; Ketone, insbesondere solche mit 3 bis 10 C-Atomen im Molekül, wie z.B. Aceton, Methylethylketon, Methyl-n-propylketon, Diethylketon, 2-Hexanon, 3-Hexanon, Di-n-propylketon, Di-isopropylke ton, Di-iso-butylketon, Cyclopentanon, Cyclohexanon, Benzophenon, Acetophenon; aliphatische Kohlenwasserstoffe, wie z.B. niedrig- und hochsiedende Petrolether; aromatische Kohlenwasserstoffe, wie z.B. Benzol, Toluol, o-, m- und p-Xylol; halogenierte aliphatische oder aromatische Kohlenwasserstoffe, wie z.B. Methylenchlorid, Chloroform, Tetrachlorkohlenstoff, Chlorbenzol, Dichlorbenzol; Nitrile, wie z.B. Acetonitril; Amide, wie z.B. Dimethylformamid, N-Methyl-pyrrolidon; Hexamethylphosphorsäuretriamid; Sulfoxide, wie z.B. Dimethylsulfoxid; Wasser. Auch Gemische verschiedener Lösungsmittel können verwendet werden.

Bei der Herstellung der Verbindungen der Formel I werden die Ausgangskomponenten normalerweise in etwa äquimolaren Mengen eingesetzt. Das Amin III kann auch in Form eines Säureadditionssalzes eingesetzt werden. Die Aufarbeitung der Ansätze erfolgt nach üblichen Methoden. Die Umsetzung kann gegebenenfalls auch in Anwesenheit einer Base oder eines Basengemischs durchgeführt werden. Geeignete Basen sind z.B.-tertiäre aliphatische Amine, wie z.B. Triethylamin, Tri-n-propylamin und Tri-iso-propylamin, ferner Pyridin, sowie Alkali-carbonate, -hydrogencarbonate, -formiate und -acetate.

Die als Ausgangsprodukte benötigten Amine der Formel III sind bekannt bzw. lassen sich leicht nach den für die Herstellung primärer Amine üblichen Methoden herstellen. Die als Ausgangsprodukte benötigten 1,4-Diketone der Formel II lassen sich, soweit sie nicht bereits bekannt sind, leicht durch katalysierte Addition eines Aldehyds der Formel IV an eine Vinyl-carbonylverbindung der Formel

$$(R^2)(R^3)N-(CH_2)_n-CHO + CH_2=CH-CO-R \longrightarrow (II)$$
$$(IV) \qquad\qquad (V)$$

nach dem Verfahren von H. Stetter, Angew. Chem. 88 (1976), 695 bis 704, herstellen, wobei in den Verbindungen IV und V die Reste R, $R^2$ und $R^3$ und n die bereits genannten Bedeutungen besitzen. Als geeignete Katalysatoren werden bei diesem Verfahren Cyanidionen in Form von Alkalimetallcyaniden oder Thiazoliumsalze, wie 5-(2-Hydroxy-ethyl)-3-benzyl-4-methyl-1,3-thiazolium-chlorid oder anderen Azoliumsalzen' in Gegenwart einer Base, wie Triethylamin oder Natriumacetat, angegeben. Die Reaktion wird ohne oder mit Lösungsmittel bei Temperaturen von 0°C bis zur Rückflußtemperatur des verwendeten Lösungsmittels durchgeführt. Es kann zweckmäßig sein, den Aldehyd der Formel IV in Form seines Hydrats einzusetzen.

Aldehyde der Formel IV können leicht durch Acylierung von Aminoacetaldehydacetalen mit Carbonsäurederivaten erhalten werden, wobei als Carbonsäurederivate besonders Carbonsäuren, Carbonsäureester, Carbonsäureanhydride und Carbonsäurechloride Verwendung finden. Die Umsetzungen werden in an sich bekannter Weise, gegebenenfalls unter Zuhilfenahme geeigneter Katalysatoren durchgeführt. Andererseits sind die Verbindungen der Formel IV auch durch Alkylierung von geeignet substituierten Aminen, Amiden oder Imiden nach literaturbekannten Verfahren zugänglich.

Aus erfindungsgemäßen Verbindungen der Formel I können die Substituenten $R^2$ und $R^3$ nach literaturbekannten Verfahren abgespalten und durch Wasserstoff ersetzt werden. Diese Abspaltung geht

besonders leicht, wenn es sich bei den Substituenten $R^2$ und $R^3$ um Acylreste, insbesondere um 1,2-Phenylendicarbonyl, Alkylendicarbonyl oder Alkenylendicarbonyl handelt. In diesen Fällen kann die Abspaltung zumeist durch Kochen mit Hydrazin bzw. Hydrazinhydrat durchgeführt werden. Man gelangt so zu erfindungsgemäßen Verbindungen der Formel I, bei der $R^2$ und $R^3$ und gegebenenfalls auch $R^1$ Wasserstoff bedeuten. Die so erhaltenen Verbindungen können durch Acylierung und/oder Alkylierung nach bekannten Methoden zu anderen erfindungsgemäßen Verbindungen der Formel I umgesetzt werden. Es ist vorteilhaft, die Reihenfolge der einzelnen Reaktionsschritte den erforderlichen Gegebenheiten anzupassen.

Falls $R^2$, $R^3$ zusammen Phenylen-1-sulfinyl-2-carbonyl oder Phenylen-1-sulfonyl-2-carbonyl bedeuten, können die Verbindungen auch durch Oxidation der entsprechenden Pheny-len-1-sulfenyl-2-carbonyl- bzw. Phenylen-1-sulfinyl-2-carbonyl-Verbindungen nach literaturbekannten Verfahren hergestellt werden.

Die Verbindungen der Formel I lassen sich vorteilhaft auch dadurch herstellen, daß bei dem vorgenannten Herstellungsverfahren das 1,4-Diketon der Formel II durch ein 1,4-Diketal der Formel IIa ersetzt wird:

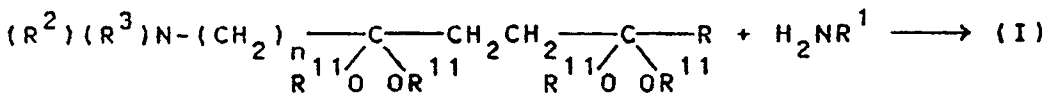

(IIa) (III)

wobei $R^{11}$ Alkyl, z.B. mit 1 bis 6 C-Atomen, und zwei benachbarte $R^{11}$ zusammen auch Alkylen, z.B. mit 2 oder 3 C-Atomen, bedeuten.

Die Umsetzungsbedingungen sind die gleichen wie bei der Umsetzung der Verbindungen II mit den Aminen III.

Die Edukte der Formel IIa erhält man aus Aminoketalen der Formel VI durch Umsetzung mit Säurederivaten AcX der Formel VII, welche den Rest $R^2CO$-, $R^3CO$- oder die für $R^2$ und $R^3$ zusammenstehenden vicinalen Phenylen, Alkylen-, Alkenylen-dicarbonyl-reste oder die für $R^2$ und $R^3$ zusammenstehenden Phenylen-1-sulfenyl- oder -sulfinyl- oder -sulfonyl-2-carbonyl-reste einführen:

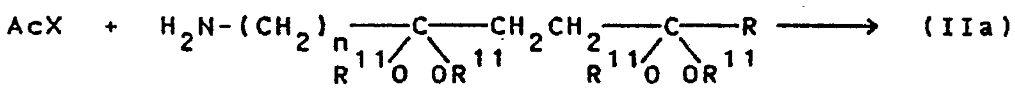

(VII) (VI)

Demgemäß hat in der Formel VII der Rest Ac die Bedeutung der vorstehend genannten Reste und AcX stellen Säurehalogenide, insbesondere Säurechloride, Säureanhydride, gemischte Sulfonsäure-carbonsäurehalogenide oder Anhydride oder andere aktivierte Säurederivate dar. Die Umsetzung kann bei Raum- oder erhöhter Temperatur, zweckmäßigerweise in einem geeigneten Lösungsmittel, z.B. einem Ether, wie z.B. Tetrahydrofuran, Dioxan, Ethylenglykoldimethylether, Diethyl- oder Dipropyl-ether, Dimethylformamid, Dimethylsulfoxid etc., gegebenenfalls in Gegenwart von Basen, wie z.B. Pyridin, Triethylamin, Kalium- oder Natriumcarbonat etc., vorgenommen werden.

Die Aminoketale der Formel VI entstehen aus leicht zugänglichen Verbindungen der Formel IIa, bei der $R^2$ und $R^3$ zusammen Phthaloyl bedeuten, durch Abspaltung des Phthalsäurerests mit Hydrazin in inerten Lösungsmitteln, wie Alkoholen, Aromaten, Ethern, Diolen, cyclischen Ethern oder aprotischen Lösungsmitteln, wie Dimethylformamid oder Dimethylsulfoxid bei Temperaturen zwischen Raumtemperatur und dem Siedepunkt des Lösungsmittels.

Die Verbindungen der Formel IIa, bei der $R^2$, $R^3$ zusammen Phthaloyl (= Phenylen-1,2-dicarbonyl) bedeuten, entstehen aus Verbindungen der Formel II nach Methoden, die zur Synthese von Ketalen in der Literatur seit langem bekannt sind (z.B. Houben-Weyl "Methoden der Org. Chemie" Bd.VI/3, (1965), Seite 217 ff; Bd;VII/2b, (1976), Seite 1886 ff). Als gut geeignet haben sich beispielsweise die Dimethylund Ethylenglykolketale erwiesen.

Verbindungen der Formel IIa, bei der $R^2$ oder $R^3$ keine Acylreste, sondern Alkylreste darstellen, lassen sich z.B. aus den Verbindungen der Formel VI durch reduktive Aminierung nach an sich bekannten Verfahren (vgl. z.B. Houben-Weyl "Methoden der Org. Chemie", Bd.11/1 (1958), Seite 641 ff) herstellen.

Sofern die 2-(Aminoalkyl)-pyrrol-Derivate der Formel I basische Reste enthalten, bilden sie mit anorganischen oder organischen Säuren Säureadditionssalze. Zur Bildung derartiger Säureadditionssalze sind anorganische und organische Säuren geeignet. Geeignete Säuren sind beispielsweise: Chlorwasserstoff, Bromwasserstoff, Naphthalindisulfonsäuren, insbesondere Naphthalin-1,5-disulfon säure, Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Nicotin-, Isonicotin-, Methansulfon-, p-Toluolsulfon-, Zitronen- oder Adipin-Säure. Pharmakologisch annehmbare Säureadditionssalze werden bevorzugt. Die Säureadditionssalze werden wie üblich durch Vereinigung der Komponenten, zweckmäßigerweise in einem geeigneten Lösungsmittel oder Verdünnungsmittel, hergestellt. Bei der Synthese der Verbindungen der Formel I können zunächst die Säureadditionssalze im Zuge der Aufarbeitung anfallen. Aus den Säureadditionssalzen können die freien Verbindungen der allgemeinen Formel I gewünschtenfalls in bekannter Weise, z.B. durch Auflösen oder Suspendieren in Wasser und Alkalischstellen, z.B. mit Natronlauge, und anschließendes Isolieren, gewonnen werden.

Die erfindungsgemäßen Verbindungen der Formel I und ihre pharmakologisch annehmbaren Säureadditionssalze besitzen wertvolle pharmakologische Eigenschaften.

Die erfindungsgemäßen Aminoalkylpyrrole der Formel I und ihre pharmakologisch annehmbaren Salze sind zentralwirksam, beispielsweise zeigen sie nootrope Wirkungen und dienen zur Behandlung von Krankheiten, die durch eine Einschränkung der Gehirnfunktion, insbesondere der Gedächtnisleistung, charakterisiert sind, sowie zur Minderung zerebraler Alterungsvorgänge. Sie sind den bisher bekannten Verbindungen gleicher Wirkungsrichtung überraschenderweise erheblich überlegen. Sie zeigen eine ausgezeichnete Wirksamkeit in verschiedenartigen Tests, wie z.B. bei der Verlängerung der Überlebenszeit unter Natriumnitrit-Hypoxie nach Gibsen und Bless (J.Neurochemistry 27, (1976)), bei der Verbesserung der stickstoffinduzierten Hypoxietoleranz, wobei Versuchstiere nach Prämedikation mit den untersuchten Präparaten mit reinem Stickstoff beatmet werden und die Verlängerung des Zeitraums zwischen Einsatz der Beatmung und elektrischer Neutralität des Elektroencephalogramms sowie die Letalität gemessen werden.

Auch in Tests, die direkt auf die Erfassung der Lern- und Gedächtnisleistung abzielen, wie z.B. den bekannten "avoidance"-Tests, sind die erfindungsgemäßen Produkte sehr gut wirksam.

Die Prüfung in den genannten und einer Reihe weiterer Tests, wie z.B. dem gamma-Butyrolacton-Test, ergibt, daß die erfindungsgemäßen Verbindungen in niedrigen Dosen bei geringer Toxizität überraschenderweise ein besonders günstiges, bei bekannten Präparaten in dieser Form nicht vorliegendes Wirkprofil aufweisen.

Die Verbindungen der Formel I und ihre physiologisch verträglichen Salze stellen somit eine Bereicherung der Pharmazie dar.

Ferner wurde gefunden, daß auch die bereits aus der Literaturstelle H.Stetter und P. Lappe, Liebigs Ann. Chem. 1980, 703 bis 714, als Zwischenprodukte bekannten Verbindungen der vorliegenden Formel I, bei denen $R^2$ und $R^3$ zusammen 1,2-Phenylen-dicarbonyl und gleichzeitig R Methyl oder Ethyl und n die Zahl 1 und $R^1$ Ethoxy-carbonylmethyl, Ethoxy-carbonyl, oder o-Methoxy-carbonyl-phenyl, oder bei denen $R^2$ und $R^3$ zusammen 1,2-Phenylen-di-carbonyl und gleichzeitig R Methyl oder Ethyl und n die Zahl 2 und $R^1$ Ethoxy-carbonyl oder Ethoxy-carbonyl-methyl, oder bei denen $R^2$ und $R^3$ zusammen 1,2-Phenylen-di-carbonyl und gleichzeitig n die Zahl 1 und R Phenyl und $R^1$ o-Methoxy-carbonyl-phenyl oder Ethoxy-carbonyl-methyl bedeuten, die gleichen, zentralwirksamen pharmakologischen Eigenschaften, wenn auch in geringerem Ausmaß, besitzen. Die erfindungsgemäßen Verbindungen der Formel I bzw. ihre pharmakologisch annehmbaren Säureadditionssalze und die vorgenannten Verbindungen können daher am Menschen als Heilmittel, z.B. bei der Bekämpfung bzw. Vorbeugung von Erkrankungen, die durch eine Einschränkung der Gehirnfunktion bedingt sind und bei der Behandlung und Vorbeugung von cerebralen Alterungsvorgängen Anwendung finden.

Die Verbindungen der Formel I und die vorgenannten Verbindungen und ihre pharmakologisch annehmbaren Säureadditionssalze können als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder einer vorgenannten Verbindung oder eines Säureadditionssalzes davon, neben üblichen pharmazeutisch einwandfreien Träger- und Zusatzstoffen enthalten. Die Zubereitungen enthalten normalerweise etwa 0,5 bis 90 Gewichtsprozent der therapeutisch wirksamen Verbindung.

Die Heilmittel können oral, z.B. in Form von Pillen, Tabletten, Lacktabletten, Dragees, Granulaten, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als

Trägerstoffe für die Herstellung von Injektionslösungen eignen sich z.B. Wasser, Alkohole, Glycerin, Polyole, pflanzliche Öle etc.

Die pharmazeutischen Präparate können neben den Wirk-und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungs-Mittel, Puffersubstanzen, ferner Lösungsmittel oder Lösungsvermittler oder Mittel zur Erzielung eines Depoteffekts, sowie Salze zur Veränderung des osmotischen Drucks, überzugsmittel oder Antioxidantien enthalten. Sie können auch zwei oder mehrere Verbindungen der Formel I oder ihrer pharmakologisch annehmbaren Säureadditionssalze und noch einen oder mehrere andere therapeutisch wirksame Stoffe enthalten.

Derartige andere therapeutisch wirksame Substanzen sind beispielsweise durchblutungsfördernde Mittel, wie Dihydroergocristin, Nicergolin, Buphenin, Nicotinsäure und ihre Ester, Pyridylcarbinol, Bencyclan, Cinnarizin, Naftidrofuryl, Raubasin und Vincamin; positiv inotrope Verbindungen, wie Digoxin, Acetyldigoxin, Metildigoxin und Lanato-Glykoside; Coronardilatatoren, wie Carbocromen, Dipyridamol, Nifedipin und Perhexilin, antianginöse Verbindungen, wie Isosorbiddinitrat, Isosorbidmononitrat, Glycerolnitrat, Molsidomin und Verapamil, β-Blocker, wie Propranolol, Oxprenolol, Atenolol, Metoprolol und Penbutolol. Darüberhinaus lassen sich die Verbindungen mit anderen nootrop wirksamen Substanzen, wie z.B. Piracetam, oder ZNSaktiven Substanzen, wie Pirlindol, Sulpirid etc. kombinieren.

Die Dosierung kann innerhalb weiter Grenzen variieren und ist in jedem einzelnen Fall den individuellen Gegebenenheiten anzupassen. Im allgemeinen ist bei oraler Verabreichung eine Tagesdosis von etwa 0,1 bis 1 mg/kg, vorzugsweise 0,3 bis 0,5 mg/kg Körpergewicht zur Erzielung wirksamer Ergebnisse angemessen, bei intravenöser Application beträgt die Tagesdosis im allgemeinen etwa 0,01 bis 0,3 mg/kg, vorzugsweise 0,05 bis 0,1 mg/kg Körpergewicht. Die Tagesdosis wird normalerweise, insbesondere bei der Applikation größerer Mengen, in mehrere, z.B. 2, 3 oder 4, Teilverabreichungen aufgeteilt. Gegebenenfalls kann es, je nach individuellem Verhalten, erforderlich werden, von der angegebenen Tagesdosis nach oben oder nach unten abzuweichen. Pharmazeutische Präparate enthalten normalerweise 0,1 bis 50 mg, vorzugsweise 0,5 bis 10 mg Wirkstoff der Formel I oder der genannten Verbindungen oder eines pharmakologisch annehmbaren Salzes pro Dosis.

1-(Ethoxy-carbonyl- bzw. Ethoxy-carbonylmethyl- bzw. o-methoxy-carbonyl-phenyl)-2-(phthalimido-methyl)-5-methyl-bzw. -ethylpyrrole sind aus der Literaturstelle H. Stetter u. P.Lappe, Liebigs Ann. Chem., 1980, 703-714, als Zwischenprodukte für die Herstellung bi- und tricyclischer Pyrrol-Systeme bekannt. Dieser Literaturstelle können jedoch keine Hinweise entnommen werden, daß die Verbindungen wertvolle pharmakologische Eigenschaften besitzen.

Von den nachfolgenden Beispielen betreffen die Beispiele 2 und 3 die Herstellung von Ausgangsprodukten, die somit keine erfindungsgemäßen Verbindungen darstellen.

**Beispiel 1**

**1-(2-Dimethylamino-ethyl)-2-(phtalimido-methyl)-5-methyl -pyrrol**

7,8g (0,03 mol) 6-Phthalimido-2,5-hexandion (vgl. Beispiel 2) und 2,7g (0,03 mol) N,N-Dimethylamino-ethylamin werden in 150 ml Essigsäure 3 h bei 80°C gerührt. Nach dem Einengen wird in wäßriger Natriumhydrogencarbonatlösung aufgenommen, mit Methylenchlorid extrahiert, getrocknet, eingeengt und aus Methanol umkristallisiert.

Ausbeute: 6,5g, 70% der Theorie; Fp: 125 bis 126°C.

Analyse:

| | | | | |
|---|---|---|---|---|
| Ber.: | C 69,4 | H 6,8 | N 13,5 | O 10,3 |
| Gef.: | C 69,1 | H 6,6 | N 13,6 | O 10,6 |

**Beispiel 2**

a) Das in Beispiel 1 benötigte Ausgangsprodukt 6-Phthal-imido-2,5-hexandion kann wie folgt hergestellt werden:. 66g (0,32 mol) Phthalimido-acetaldehydhydrat (vgl. Beispiel 3), 70,1g (1,0 mol) Methylvinylketon, 31,5g 3-Ben-zyl-5-(2-hydroxyethyl)-4-methyl-1,3-thiazoliumchlorid und 155ml Triethylamin werden in 600ml Ethanol 16 h unter Rückfluß erhitzt. Nach dem Einengen wird in wäßriger Natriumhydrogencarbonatlösung aufgenommen, mit Methylenchlorid extrahiert, über Aktivkohle filtriert, eingeengt und aus Methanol umkristallisiert.

Asbeute: 55,1g, 67% der Theorie; Fp: 117 bis 119°C.

Analyse:

| | | | | |
|---|---|---|---|---|
| Ber.: | C 64,9 | H 5,1 | N 5,4 | O 24,7 |
| Gef.: | C 64,8 | H 5,4 | N 5,5 | O 24,7 |

In ähnlicher Weise lassen sich beispielsweise herstellen:

b) 6-Succinimido-hexan-2,5-dion
c) 6-Maleinimido-hexan-2,5-dion
d) 6-Acetamido-hexan-2,5-dion
e) 6-(N,N-Dimethylamino)-hexan-2,5-dion
f) 6-Benzamido-hexan-2,5-dion
g) 6-(4-Methylbenzamido)-hexan-2,5-dion
h) 6-(4°Chlorbenzamido)-hexan-2,5-dion
i) 6-(4-Methoxybenzamido)-hexan-2,5-dion
j) 6-(3,4-Dimethoxybenzamido)-hexan-2,5-dion
k) 6-(Nicotinamido)-hexan-2,5-dion
l) 6-(Pyridin-2,3-dicarboximido)-hexan-2,5-dion
m) 6-(o-sulfobenzoesäureimido)-hexan-2,5-dion

**Beispiel 3**

Das in Beispiel 2 benötigte Ausgangsprodukt Phthalimidoacetaldehydhydrat kann nach den Lehren des deutschen Bundespatents 982 711 wie folgt hergestellt werden:

a) 500g Acetamid werden im Ölbad geschmolzen. Nach Zugabe von 243,5g (1,31 mol) Phthalimidkalium wird auf 130°C erwärmt und bei dieser Temperatur 259g (1,31 mol) Bromacetaldehyd-diethylacetal zugegeben. Nach beendeter Zugabe wird 30 min auf 170°C erwärmt, abgekühlt, mit 1 l Wasser versetzt und über Nacht gerührt. Der ausgefallene Feststoff wird abgesaugt, mit Methylenchlorid ausgerührt, die Methylenchloridphase getrocknet und eingeengt.

Ausbeute: 256,6g Phthalimido-acetaldehyd-diethylacetal = 74% der Theorie, Fp: 70 bis 71°C.

256,6g (0,975 mol) Phthalimido-acetaldehyd-diethylacetal, 270ml Ameisensäure, 25ml konzentrierte Salzsäure und 40ml Wasser werden 10 min lang auf 100°C erwärmt, nach dem Abkühlen mit 3 l Wasser verdünnt und auf 2 l Lösung eingeengt und abgekühlt. Das ausgefallene Produkt wird abgesaugt und getrocknet.

Ausbeute: 162g = 80% der Theorie Phthalimido-acetaldehydhydrat vom Fp: 112 bis 113°C.

Bei dem vorstehenden Verfahren kann anstelle von Bromacetaldehyd-diethylacetal mit ähnlich gutem Erfolg auch Chloracetaldehyd-diethylacetal eingesetzt werden.

b) 100g (0,54 mol) Phthalimidkalium, 90g (0,46 mol) Bromacetaldehyd-diethylacetal und 1g Kaliumjodid werden in 800ml Dimethylformamid 66 h bei 100°C gerührt. Nach dem Einengen auf 100ml wird mit 200ml Wasser versetzt, abgesaugt und mit 145ml Ameisensäure, 20ml Wasser und 15ml konzentrierte Salzsäure versetzt, 10 min auf 100°C erhitzt, 1 l Wasser zugegeben, über Aktivkohle filtriert, abgekühlt, abgesaugt und getrocknet.

Ausbeute: 62,4g = 66% der Theorie Phthalimido-acetaldehydhydrat vom Fp: 111 bis 113°C.

Nach den Verfahren a) und b) lassen sich in analoger Weise z.B. herstellen: Pyridin-2,3-dicarboximido-acetaldehyd und -acetaldehyddiethylacetal, Succinimido-acetaldehyd-diethylacetal, Succinimido-acetaldehyd, Maleinimido-acetaldehyd-diethylacetal, Maleinimido-acetaldehyd, (1,2-Benzisothiazol-1,1-dioxid-3(2H)-on-2-yl)-acetaldehyd und -acetaldehyd-diethylacetal (=o-Sulfobenzoesäureimido-acetaldehyd und o-Sulfobenzoesäureimido-acetaldehydacetal).

**Beispiel 4**

13,0g (0 05 mol) 6-Phthalimido-2,5-hexandion und 9,1g (0,05 mol) 2-(3,4-Dimethoxyphenyl)-ethylamin werden in 250ml Essigsäure 7 h bei 80°C gerührt. Nach Aufarbeitung wie in Beispiel 1 erhält man 7,3g Produkt.

Ausbeute: 36 % der Theorie; Fp: 112 bis 113°C.

| Analyse: | | | | |
|---|---|---|---|---|
| Ber.: | C 71,3 | H 6,0 | N 6,9 | O 15,8 |
| Gef.: | C 70,9 | H 6,0 | N 6,8 | O 16,1 |

**Beispiel 5**

**1-(Methoxycaarbonyl-methyl)-2-(phthalimido-methyl)-5-methyl-pyrrol**

18,1g (0,07 mol) 6-Phthalimido-2,5-hexandion, 10,0g (0,08 mol) Glycinmethylesterhydrochlorid und 6,6g (0,08 mol) Natriumacetat werden in 300ml Essigsäure 3 h bei 80°C gerührt. Nach dem Einengen wird in wäßriger Natriumhydrogen-carbonatlösung aufgenommen, mit Methylenchlorid extrahiert, getrocknet, eingeengt und aus Methanol umkristallisiert.

Ausbeute: 14,5g, 66% der Theorie, Fp: 134°C.

| Analyse: | | | | |
|---|---|---|---|---|
| Ber.: | C 65,4 | H 5,2 | N 9,0 | O 20,5 |
| Gef.: | C 65,1 | H 5,1 | N 9,0 | O 20,8 |

**Beispiel 6**

**1-(2-Methoxy-carbonyl-4,5-dimethoxy-phelyl)-2-(phthalimido-methyl)-5-methyl-pyrrol**

13,0g (0,05 mol) 6-Phthalimido-2,5-hexandion und 10,6g (0,05 mol) 2-Amino-4,5-dimethoxybenzoesäure-methylester werden in 250ml Essigsäure 30 h bei 100°C gerührt und wie in Beispiel 1 aufgearbeitet.

Ausbeute: 10,3g, 47% der Theorie, Fp: 184 bis 185°C.

Analyse:

| | | | | |
|---|---|---|---|---|
| Ber.: | C 66,4 | H 5,1 | N 6,4 | O 22.1 |
| Gef.: | C 66,4 | H 5,0 | N 6,6 | O 22,5 |

**Beispiel 7**

**2-(Phthalimido-methyl)-5-methyl-pyrrol**

36,3g (0,14 mol) 6-Phthalimido-2,5-hexandion und 54g (0,7 mol) Ammoniumacetat und 1g Ammoniumchlorid werden in 700ml Essigsäure 1 h bei 80° C gerührt und wie in Beispiel 1 aufgearbeitet.
Ausbeute: 32,2g, 96% der Theorie, Fp: 173 bis 174° C.

Analyse:

| | | | | |
|---|---|---|---|---|
| Ber.: | C 70,0 | H 5,0 | N 11,7 | O 13,3 |
| Gef.: | C 69,7 | H 5,3 | N 11,4 | O 13,8 |

**Beispiel 8**

**1-(2-(Imidazol-4(5)-yl)-ethyl)-2-(phthalimido-methyl)-5 methyl-pyrrol-hydrochlorid**

5,2g (0,02 mol) 6-Phthalimido-2,5-hexandion und 3,7g (0,02mol) Histamindihydrochlorid und 3,4g (0,04 mol) Natriumacetat werden in 70ml Essigsäure 3 h bei 80°C gerührt. Nach der Aufarbeitung wie in Beispiel 1 wird mit ethanolischer Salzsäure versetzt, abfiltriert und getrocknet.
Ausbeute: 4,0g, 54% der Theorie, Fp: 218 bis 219° C.

Analyse:

| | | | | | |
|---|---|---|---|---|---|
| Ber.: | C 61,5 | H 5,2 | N 15,1 | O 8,6 | Cl 9,6 |
| Gef.: | C 61,2 | H 5,3 | N 14,8 | O 9,0 | Cl 9,4 |

**Beispiel 9**

**1-(Methoxycarbonyl-methyl)-2-(phthalimido-methyl)-5 methyl-pyrrol**

11,4g (0 044 mol) 6-Phthalimido-2,5-hexandion und 27,6g (0,22 mol) Glycinmethylester-hydrochlorid und 9,1g (0,11 mol) Natriumacetat werden in Ethanol 16 h unter Rückfluß erhitzt und wie in Beispiel 1 aufgearbeitet.
Ausbeute: 6,1g, 44% der Theorie, Fp: 134 bis 135°C.

Analyse:

| | | | | |
|---|---|---|---|---|
| Ber.: | C 65,4 | H 5,2 | N 9,0 | O 20,5 |
| Gef.: | C 65,7 | H 5,2 | N 9,0 | O 20,1 |

**Beispiel 10**

**1-(2-Acetylamino-ethyl)-2-(phthalimido-methyl)-5-methylpyrrol**

4,6g (0,018 mol) 6-Phthalimido-2,5-hexandion und 1,8g (0,018 mol) 2-(Acetylamino)-ethylamin werden in 120ml Essigsäure 4 h bei 80°C gerührt. Die Aufarbeitung erfolgt wie in Beispiel 1 angegeben:
Ausbeute: 1,5g, 26% der Theorie, Fp: 191 bis 192°C

Analyse:

| | | | | |
|---|---|---|---|---|
| Ber.: | C 66,4 | H 5,9 | N 13,2 | O 14,8 |
| Gef.: | C 66,2 | H 6,0 | N 12,9 | O 14,9 |

**Beispiel 11**

**2-(Amino-methyl)-5-methyl-pyrrol**

60,9g (0,23 mol) 2-(Phthalimido-methyl)-5-methyl-pyrrol (vgl. Beispiel 7) und 114ml (2,3 mol) Hydrazinhydrat werden in 1 l Ethanol 15 min unter Rückfluß erhitzt. Nach dem Erkalten wird abgesaugt; eingeengt und der Rückstand in Methylenchlorid aufgenommen, abfiltriert, das Filtrat eingeengt und aus Toluol umkristallisiert.
Ausbeute: 23,1g, 91% der Theorie.

**Beispiel 12**

**1-(2-(3,4-Dimethoxyphenyl)-ethyl)-2-(amino-methyl) -5methyl-pyrrol**

6,0g (0,015 mol) 1-(2-(3,4-Dimethoxyphenyl)-ethyl)-2-(phthalimido-methyl)-5-methyl-pyrrol (vgl. Beispiel 4) und 7ml (0,15 mol) Hydrazinhydrat werden 30 min unter Rückfluß erhitzt. Nach dem Einengen wird in Methylenchlorid aufgenommen, abfiltriert, eingeengt und über eine Kieselgelsäule chromatographiert.
Ausbeute: 3,0g, 74 % der Theorie.

**Beispiel 13**

**1-(2-Dimethylamino-ethyl)-2-(amino-methyl)-5-methyl-pyr rol**

5,0g (0,014 mol) 1-(2-Dimethylamino-ethyl)-2-(phthal imidomethyl)-5-methyl-pyrrol (vgl. Beispiel 1) und 7 ml (0,15 mol) Hydrazinhydrat werden 30 min unter Rückfluß erhitzt. Nach dem Abkühlen wird vom Phthalsäurehydrazid abgesaugt, eingeengt, in Methylenchlorid aufgenommen, nochmals filtriert und eingeengt.
Ausbeute: 2,4g, 94% der Theorie.

Analyse:

| | | | |
|---|---|---|---|
| Ber.: | C 66,3 | H 10,6 | N 23,2 |
| Gef.: | C 66,5 | H 10,1 | N 22,8 |

**Beispiel 14**

**1-(2-Dimethylamino-ethyl)-2-(3,4-dimethoxy-benzoylaminomethyl-5-methyl-pyrrol**

2,3g (0,013 mol) 3,4-Dimethoxybenzoesäure werden mit 2,1g (0,013 mol) Carbonyldiimidazol in 40ml Tetrahydrofuran 15 min unter Rückfluß erhitzt. Nach Zugabe von 2,3g (0 013 mol) 1-(2-(Dimethylamino)-ethyl)-2-(aminomethyl)-5-methyl-pyrrol (vgl.Beispiel 12) wird 3 h bei Raumtemperatur gerührt, eingeengt, mit wäßriger Natriumhydrogen-carbonatlösung versetzt, mit Methylenchlorid extrahiert, getrocknet, eingeengt und über eine Kieselgelsäule chromatographiert.
Ausbeute: 1,8g, 41% der Theorie.

**Beispiel 15**

**1-(2-(3,4-Dimethoxyphenyl)-ethyl)-2-(pyridyl-3-carbonyl amino-methyl)-5-methyl-pyrrol**

1,4g (0,011 mol) Pyridin-3-carbonsäure und 1,8g (0,011 mol) Carbonyldiimidazol werden in 40ml Tetrahydrofuran 15 min unter Rückfluß erhitzt. Nach Zugabe von 3,0g (0 011 mol) 1-(2-(3,4-Dimethoxyphenyl)-ethyl)-2-(aminomethyl)-5-methyl-pyrrol (vgl. Beispiel 12) wird 2 h bei Raumtemperatur gerührt, eingeengt, mit wäßriger Natriumhydrogen-carbonatlösung versetzt, mit Methylenchlorid extrahiert, getrocknet, eingeengt und aus Isopropanol umkristallisiert.
Ausbeute: 1,9g, 46% der Theorie, Fp: 116 bis 117° C.

Analyse:

| | | | | |
|---|---|---|---|---|
| Ber.: | C 69,6 | H 6,6 | N 11,1 | O 12,6 |
| Gef.: | C 69,8 | H 6,5 | N 11,0 | O 12,6 |

**Beispiel 16**

**2-(3,4-Dimethoxy-benzoylamilo-methyl)-5-methyl-pyrrol**

18,1g (0,1 mol) 3,4-Dimethoxybenzoesäure werden mit 17,8g (0,11 mol) Carbonyldiimidazol und 11,0g (0,1 mol) 2-Ami-nomethyl-5-methyl-pyrrol (vgl. Beispiel 11), wie in Beispiel 15 beschrieben, umgesetzt.
Ausbeute: 24,4g, -9% der Theorie, Fp: 125 bis 126° C.

Analyse:

| | | | | |
|---|---|---|---|---|
| Ber.: | C 65,7 | H 6,6 | N 17,5 | O 10,2 |
| Gef.: | C 65,8 | H 6,6 | N 17,4 | O 10,1 |

**Beispiel 17**

**2-(Pyridyl-3-carbonylamino-methyl)-5-methyl-pyrrol**

12,9g (0,1 mol) Pyridin-3-carbonsäure, 17,8g (0,11 mol) Carbonyldiimidazol und 11,0g (0,1 mol) 2-(Aminomethyl)-5-methyl-pyrrol (vgl. Beispiel 11), werden wie in Beispiel 15 beschrieben, umgesetzt.
Ausbeute: 14,1g, 66% der Theorie, Fp: 157 bis 158° C.

Analyse:

| | | | | |
|---|---|---|---|---|
| Ber.: | C 67,0 | H 6,1 | N 19,5 | O 7,4 |
| Gef.: | C 66,7 | H 6,0 | N 19,5 | O 7,6 |

**Beispiel 18**

**2-(Acetylamino-methyl)-5-methyl-pyrrol**

Zu 6,4g (0,06 mol) 2-(Amino-methyl)-5-methyl-pyrrol (vgl. Beispiel 11) in 20ml Methylenchlorid werden bei 10°C 6,0g (0,06 mol) Essigsäureanhydrid in 20ml Methylenchlorid zugetropft. Nach 2 h Reaktionszeit bei Raumtemperatur wird eingeengt und über eine Kieselgelsäure chromatographiert.

Ausbeute: 5,2g, 59% der Theorie,

**Beispiel 19**

**1-(2-Cyanoethyl)-2-(3,4-dimethoxybenzoylamino-methyl)-5methyl-pyrrol**

Zu 5,1g (0,019 mol) 2-(3,4-Dimethoxybenzoylamino-methyl)-5-methyl-pyrrol (vgl. Beispiel 16) und 15 ml (0,23 mol) Acrylsäurenitril werden unter Kühlung 3 ml Benzyltrimethyl-ammoniumhydroxid (40%ige Lösung in Methanol) zugetropft. Nach 30 min wird mit Methylenchlorid versetzt, abfiltriert, Wasser zugegeben, die Methylenchloridphase abgetrennt und eingeengt. Umkristallisation aus Essigester ergibt 4,3g Produkt vom Fp: 175 bis 176° C.

Ausbeute: 4,3g, 71% der Theorie.

Analyse:

| | | | | |
|---|---|---|---|---|
| Ber.: | C 66,0 | H 6,5 | N 12,8 | O 14,7 |
| Gef.: | C 65,7 | H 6,2 | N 12,6 | O 15,1 |

**Beispiel 20**

**1-(Aminocarbonyl-methyl-2-(phthal-imido-methyl)-5-Methylpyrrol**

5,2g (0,02 mol) 6-Phthalimido-2,5-hexandion, 2,3g (0,02 mol) Glycinamidhydrochlorid und 1,7g (0,02 mol) Natriumacetat werden in 70 ml Essigsäure 3 h bei 80° C gerührt und wie in Beispiel 1 aufgearbeitet.

Ausbeute: 2,3g, 39% der Theorie, Fp.: 169 bis 171° C

Analyse:

| | | | | |
|---|---|---|---|---|
| Ber.: | C 64.6 | H 5,1 | N 14,1 | O 16,1 |
| Gef.: | C 64,3 | H 5,2 | N 14,2 | O 16,3 |

**Beispiel 21**

**1-(3-(1-Imidazolyl)-Propyl)-2-(phtalimido-methyl)-5methyl-pyrrol-hydrochlorid**

5,2g (0,02 mol) 6-Phthalimido-2,5-hexandion und 2,5g (0,02 mol) 3-(1-Imidazolyl)-propylamin werden in 70m1 Essigsäure 2 h bei 80°C gerührt und wie in Beispiel 1 aufgearbeitet. Nach Versetzen mit ethanolischer Salzsäure wird abfiltriert und aus Ethanol umkristallisiert.

Ausbeute: 1,1g, 14% der Theorie, Fp.: 217 bis 218°C

Analyse:

| | | | | |
|---|---|---|---|---|
| Ber.: | C 62,4 | H 5,5 | N 14,6 | O 8,3 |
| Gef.: | C 62,3 | H 5,7 | N 14,9 | O 8,6 |

Die nachfolgenden Beispiele 22 bis 26 betreffen die Herstellung von Edukten der Formel IIa, die Beispiele 27 bis 38 und die Beispiele der Tabellen I bis III betreffen weitere erfindungsgemäße Verbindugen.

**Beispiel 22**

**6-Phthalimido-2,5-hexandion-bis-(dimethylketal)**

259 3 g (1,0 mol) 6-Phthalimido-2,5-hexandion (vgl. Beispiel 2 und 318,4 g (3,0 mol) o-Ameisensäuretrimethylester werden in 320 ml Methanol und 20 ml methanolischer Salzsäure 3 h bei Feuchtigkeitsausschluß unter Rückfluß erhitzt. Nach der Neutralisation mit Piperidin wird im Vakuum eingeengt.

Ausbeute: 351 g (99,9 % der Theorie), Fp: 73 bis 75°C.

| | | | | |
|---|---|---|---|---|
| Ber.: | C 62,8 | H 6,9 | N 3,9 | O 26,4 |
| Gef.: | 62,5 | 7,1 | 3,9 | 26,5 |

**Beispiel 23**

**6-Amino-2,5-hexandion-bis-(dimethylketal)**

501,5 g (1,43 mol) 6-Phthalimido-2,5-hexandion-bis-(di-methylketal) (vgl. Beispiel 22) und 500 ml (10,3 mol) Hydrazinhydrat werden in 6 l wasserfreiem Ethanol 15 min unter Rückfluß erhitzt. Nach dem Abfiltrieren des ausgefallenen Phthalsäurehydrazids wird im Vakuum eingeengt, in Methylenchlorid aufgenommen, getrocknet und abfiltriert und eingeengt.

Ausbeute: 250,5 g (79 % der Theorie)

Das erhaltene Öl kann direkt weiter umgesetzt werden.

**Beispiel 24**

**6-(3,4-Dimethoxybenzoyl)-amino-2,5-hexandion-bis-(dimethylketal)**

18,2 g (0,1 mol) 3,4-Dimethoxybenzoesäure und 16,2 g (0,1 mol) Carbonyldiimidazol werden in 150 ml wasserfreiem Ethylenglykoldimethylether 15 min unter Rückfluß erhitzt. Nach dem Abkühlen werden 22,1 g (0,1 mol) 6-Amino-2,5-hexandion-bis-(dimethylketal) (vgl. Beispiel 23) zugegeben. Nach 2 h Reaktionszeit bei Raumtemperatur wird im Vakuum eingeengt, in Methylenchlorid aufgenommen, mit Natriumhydrogencarbonatlösung gewaschen, getrocknet und eingeengt.

Ausbeute: 33,7 g (87 % der Theorie), Fp: 92 bis 93° C.

| | | | | |
|---|---|---|---|---|
| Ber.: | C 59,2 | H 8,1 | N 3,6 | O 29,1 |
| Gef.: | 59,4 | 7,9 | 3,4 | 29,2 |

**Beispiel 25**

**6-Acetylamino-2,5-hexandion-bis-(dimethylketal)**

Zu 44,2 g (0,2 mol) 6-Amino-2,5-hexandion-bis-(dimethylketal) in 70 ml Methylenchlorid werden bei 10°C 20,6 g (0,2 mol) Essigsäureanhydrid in 70 ml Methylenchlorid zugetropft. Nach 2 h bei 10°C läßt man auf Raumtemperatur erwärmen, wäscht mit Natriumhydrogencarbonatlösung, trocknet und engt ein.

Ausbeute: 50,2 g (95 % der Theorie)

Das erhaltene Öl wird direkt weiter umgesetzt.

**Beispiel 26**

**6-(Phenyl-1-sulfenyl-2-carbonyl)-imino-2,5-hexandion-bis(dimethylketal)**

Zu 8,8 g (0,04 mol) 6-Amino-2,5-hexandion-bis-(dimethylketal) und 20 ml Triethylamin in 50 ml Toluol werden langsam 8,3 g (0,04 mol) Benzol-1-sulfensäure-2-carbon-säuredichlorid in 17 ml Toluol zugetropft. Nach 3 h Reaktionszeit wird eingeengt, in Methylenchlorid gelöst, mit Natriumhydrogencarbonatlösung gewaschen, getrocknet, eingeengt und mit Diethylether verrührt.

Ausbeute: 7,4 g (52 % der Theorie), Fp: 115 bis 116° C.

In analoger Weise lassen sich beispielsweise herstellen:

6-(4-Methoxybenzoyl)-amino-2,5-hexandion-bis-(dimethylketal)
6-(Pyridyl-3-carbonyl)-amino-2,5-hexandion-bis-(dimethylketal)
6-(4-Chlorbenzoyl)-amino-2,5-hexandion-bis-(dimethylketal),
6-(1-Acetyl-pyrrolidin-2-carbonyl)-amino-2,5-hexandion-bis-(dimethylketal),
6-(3,4-Dichlorphenyl-1-amino-carbonyl)-amino-2,5-hexan-dion-bis-(dimethylketal),
6-(4-Acetyloxy-3-methoxy-benzoyl)-amino-2,5-hexandion-bis-(dimethylketal),
6-(4-Chlorphenoxyacetyl)-amino-2,5-hexandion-bis-(dimethylketal),
6-(4-Chlorphenoxyacetyl)-amino-2,5-hexandion-bis-(ethylenglykolketal),
6-(4-Methylbenzoyl)-amino-2,5-hexandion-bis-(dimethylketal),
6-(2-Methoxycarbonylphenyl-1-sulfonyl)-amino-2,5-hexandion-bis-(dimethylketal),
6-(3-Acetyloxy-2-phenyl-propionyl)-amino-2,5-hexandion-bis-(dimethylketal),
6-(2-Acetyloxy-2-phenyl-acetyl)-amino-2,5-hexandion-bis-(dimethylketal),
6-(4-Nitrobenzoyl)-amino-2,5-hexandion-bis-(dimethylketal),
6-(4-Aminobenzoyl)-amino-2,5-hexandion-bis-(dimethylketal),
6-(4-Methylphenyl-1-sulfonyl)-amino-2,5-hexandion-bis-(dimethylketal),
6-(3-Hydroxy-2-phenyl-propionyl)-amino-2,5-hexandion-bis-(dimethylketal),

6-(2-Hydroxy-2-phenyl-acetyl)-amino-2,5-hexandion-bis-(dimethylketal),
6-(1-Formyl-pyrrolidon-3-carbonyl)-amino-2,5-hexandion-bis-(dimethylketal),
7-Phthalimido-3,6-heptandion-bis-(dimethylketal)
5-Phthalimido-1-phenyl-1,4-pentan-dion-bis-(dimethylketal),
7-Phthalimido-2,5-hexandion-bis-(dimethylketal),
8-Phthalimido-2,5-octandion-bis-(dimethylketal),
6-Succinimido-2,5-hexandion-bis-(ethylenglykolketal)
6-Maleinimido-2,5-hexandion-bis-(ethylenglykolketal)
6-(Phenylen-2,3-dicarbonyl)-imino-2,5-hexandion-bis-(dimethylketal),
6-(Phenylen-2-carbonyl-1-sulfonyl)-imino-2,5-hexandion-bis-(dimethylketal),
6-(Phenylen-2-carbonyl-1-sulfinyl)-imino-2,5-hexandion-bis-(dimethylketal).
Die erhaltenen Verbindungen können zumeist in Form der Rohprodukte weiterverarbeitet werden.

**Beispiel 27**

**1-(2-Acetylamino-ethyl)-2-(4-methoxybenzoyl)-aminomethyl-5-methyl-pyrrol**

32 g (0,09 mol) 6-(4-Methoxybenzoyl)-amino-2,5-hexandion-bis-(dimethylketal) und 9,7 g(0,095 mol) 1-Acetylamino-ethylendiamin werden in 200 ml Essigsäure 12 h bei 40°C gerührt. Nach Einengen im Vakuum wird in Methylenchlorid aufgenommen, mit Natriumhydrogencarbonatlösung gewaschen, getrocknet, eingeengt und mit Diethylether verrührt.

Ausbeute: 7,4 g (25 % der Theorie), Fp. 150 bis 151°C

| | | | | |
|---|---|---|---|---|
| Ber.: | C 65,6 | H 7,0 | N 12,8 | O 14,6 |
| Gef.: | 65,7 | 7,0 | 12,5 | 14,5 |

**Beispiel 28**

**1-(Methoxycarbonyl-methyl)-2-(phthalimido-methyl)-5 -phenyl-pyrrol**

9,6 g (0,03 mol) 5-Phthalimido-1-phenyl-1,4-pentandion, 5,0 g (0 04 mol) Glycinmethylester-hydrochlorid und 3,2 g (0,04 mol) Natriumacetat wasserfrei werden in 80 ml Propionsäure 20 h bei 60°C gerührt. Reinigung durch Säulenchromatographie. Feste Phase: "Silica-Woelm", Korngröße 0,1 bis 0,2 mm; Hersteller Fa. Woelm, Eschwege, Deutschland; Laufmittel: Methylenchlorid.

Ausbeute: 3,9 g (35 % der Theorie), Fp. 160 bis 162°C

| | | | | |
|---|---|---|---|---|
| Ber.: | C 70,6 | H 4,8 | N 7,5 | O 17,1 |
| Gef.: | 70,4 | 5,0 | 7,5 | 17,2 |

**Beispiel 29**

**1-(2-(3-Indolyl)-1-methoxycarbonyl)-ethyl-2-(phtalimidomethyl)-5-methyl-pyrrol**

3 9 g (0 015 mol) 6-Phthalimido-2,5-hexandion, 4,0 g (0,016 mol) Tryptophanmethylester-hydrochlorid und 2ml (0,014 mol) Tricthylamin werden in 80 ml Ethylenglykolmonomethylether 6 h bei 50°C gerührt und wie in Beispiel 27 aufgearbeitet.

Ausbeute: 3,5 g (53 % der Theorie), Fp. 168 bis 169°C

| | | | | |
|---|---|---|---|---|
| Ber.: | C 70,7 | H 5,3 | N 9,5 | O 14,5 |
| Gef.: | 71,0 | 5,2 | 9,4 | 14,7 |

**Beispiel 30**

**1-(4,5-dimethoxy-2-methoxycarbonyl-phenyl)-2-(4-chlor phenoxyacetyl-amino-methyl)-5-methyl-pyrrol**

9,7 g (0,025 mol) 6-(4-Chlorphenoxyacetyl)-amino-2,5-hexandion-bis-(dimethylketal) und 5,3 g 4,5-Dimethoxy-anthranilsäure werden in 100 ml Essigsäure 8 h bei 50°C gerührt und wie in Beispiel 27 aufgearbeitet.

Ausbeute: 2,2 g (19 % der Theorie), Fp. 92 bis 93°C

| | | | | | |
|---|---|---|---|---|---|
| Ber.: | C 61,0 | H 5,3 | Cl 7,5 | N 5,9 | O 20,3 |
| Gef.: | 60,5 | 5,4 | 7,8 | 5,8 | 20,4 |

**Beispiel 31**

**1-(Methoxycarbonyl-methyl)-2-(2-phthalimido-ethyl)-5methyl-pyrrol**

82 g (0,03 mol) 7-Phthalimido-2,5-heptandion, 5,0 g (0,04 mol) Glycinmethylester-hydrochlorid und 3,3 g (0,04 mol Natriumacetat werden in 150 ml Ameisensäure 4 h bei 60°C gerührt und wie in Beispiel 27 aufgearbeitet.

Ausbeute: 2,9 g (30 % der Theorie), Fp. 125 bis 128° C

| | | | | |
|---|---|---|---|---|
| Ber.: | C 66,2 | H 5,6 | N 8,6 | O 19,6 |
| Gef.: | 66,0 | 5,6 | 9,0 | 19,2 |

**Beispiel 32**

**1-(2-(Ethoxycarbonyl)-ethyl)-2-(phtalimido-methyl)-5-methyl-pyrrol**

3,9 g (0,015 mol) 6-Phthalimido-2,5-hexandion, 3,1 g (0,02 mol) β-Alaninethylester-hydrochlorid und 3 ml (0,02 mol) Triethylamin werden in 80 ml Eisessig 8 h bei 50° C gerührt und wie in Beispiel 27 aufgearbeitet.

Ausbeute: 2,1 g (41 % der Theorie), Fp. 106 bis 108° C

| | | | | |
|---|---|---|---|---|
| Ber.: | C 67,0 | H 5,9 | N 8,2 | O 18,8 |
| Gef.: | 67,3 | 5,9 | 8,5 | 18,7 |

**Beispiel 33**

**1-(3-Ethoxycarbonyl)-propyl)-2-(phthalimido-methyl)-5 -methyl-pyrrol**

3,9 g (0 015 mol) 6-Phthalimido-2,5-hexandion, 2,5 g (0,015 mol) 4-Aminobuttersäureethylester-hydrochlorid und 1,3 g (0,015 mol) Natriumacetat werden 23 h bei 60° C in 50 ml Toluol gerührt. Nach dem Einengen wird wie in Beispiel 22 aufgearbeitet.

Ausbeute: 2,7 g (51 % der Theorie) Fp. 92 bis 94° C

| | | | | |
|---|---|---|---|---|
| Ber.: | C 67,8 | H 6,3 | N 7,9 | O 18,1 |
| Gef.: | 67,2 | H 6,3 | 8,1 | 17,7 |

**Beispiel 34**

**1-(Methoxycarbonyl-methyl)-2-(phthalimido-methyl)-5-ethyl-pyrrol**

6,8 g (0,025 mol) 7-Phthalimido-3,6-heptandion, 3,5 g (0,02- mol) Glycinmethylester-hydrochlorid, und 2,3 g (0,028 mol) Natriumacetat werden in 150 ml Cyclohexanol 13 h bei 60°C gerührt und wie in Beispiel 22 aufgearbeitet.

Ausbeute: 5,4 g (66 % der Theorie), Fp. 94 bis 95°C

| | | | | |
|---|---|---|---|---|
| Ber.: | C 66,2 | H 5,6 | N 8,6 | O 19,6 |
| Gef.: | 66,3 | 5,6 | 8,8 | 19,7 |

**Beispiel 35**

**1-(2-Methoxy-carbonyl-4,5,6-trimethoxy-phenyl)-2(phthalimido-methyl)-5-methyl-pyrrol**

7,8 g (0,03 mol) 6-Phthalimido-2,5-hexandion und 7,3 g (0,03 mol) 2-Amino-3,4,5-trimethoxybenzoesäuremethylester werden in 120 ml Dimethylformamid 16 h bei 80°C gerührt und wie in Beispiel 27 aufgearbeitet.

Ausbeute: 3,5 g (25 % der Theorie), Fp. 146 bis 148°C

| | | | | |
|---|---|---|---|---|
| Ber.: | C 64,6 | H 5,2 | N 6,0 | O 24,1 |
| Gef.: | 64,0 | 5,3 | 6,3 | 24,3 |

**Beispiel 36**

**1-(Hydroxy-carbonyl-methyl)-2-(phthalimido-methyl)-5-methyl-pyrrol**

5,2 g (0,02 mol) 6-Phthalimido-2,5-hexandion und 1,6 (0,02 mol) Glycin werden 1 h bei 80°C in 50 ml Acetonitril gerührt. Nach dem Einengen wird alkalisch mit Methylenchlorid extrahiert, angesäuert und nochmals extrahiert. Das er altene Produkt wird aus Methanol umkristallisiert.

Ausbeute: 2,8 g (47 % der Theorie), Fp. 154 bis 157° C

| | | | | |
|---|---|---|---|---|
| Ber.: | C 64,4 | H 4,7 | N 9,4 | O 21,5 |
| Gef.: | 64,3 | H 5,1 | 9,2 | 21,3 |

**Beispiel 37**

**1-Cyanomethyl-2-(phthalimido-methyl)-5-methyl-pyrrol**

10,4 g (0,04 mol) 6-Phthalimido-2,5-hexandion, 6,2 g (0,04 mol) Aminoacetonitril-sulfat und 6,8 g (0,08 mol) Natriumacetat werden in 200 ml Ethylenglykoldimethylether 16 h bei 80° C gerührt und wie in Beispiel 27 aufgearbeitet.
Ausbeute: 5,2 g (47 % der Theorie), Fp. 166 bis 168° C

| | | | | |
|---|---|---|---|---|
| Ber.: | C 68,8 | H 4,7 | N 15,0 | O 11,5 |
| Gef.: | 68,1 | H 5,0 | 14,8 | 11,9 |

**Beispiel 38**

**1-(tert-Butoxy-carbonyl-methyl)-2-(phthalimido-methyl)-5-methyl-pyrrol**

13,0 g (0,05 mol) 6-Phthalimido-2,5-hexandion und 6,6 g (0,05 mol) Glycin-tert-butylester werden in 250 ml Tetrahydrofuran 20 h bei 80° C gerührt und wie in Beispiel 27 aufgearbeitet.
Ausbeute: 8,7 g (49 % der Theorie), Fp. 134 bis 135° C

| | | | | |
|---|---|---|---|---|
| Ber.: | C 67,8 | H 6,3 | N 7,9 | O 18,1 |
| Gef.: | 68,1 | H 6,3 | 7,6 | 18,3 |

Weitere Beispiele von hergestellten Verbindungen sind in den folgenden Tabellen I bis III aufgeführt.

**TABELLE I**

Verbindungen der Formel I mit R = $CH_3$, $R^2$ = H und n = 1 und

| $R^1$ | $R^3$ | Fp: (°C) |
|---|---|---|
| $-CH_2COOCH_3$ | 3,4-Dimethoxy-benzoyl | 138 - 139 |
| $-CH_2CH_2COOC_2H_5$ | 3,4-Dimethoxy-benzoyl | 147 - 148 |
| $-CH_2CH_2CH_2COOC_2H_5$ | 3,4-Dimethoxy-benzoyl | 96 - 98 |
| $-CH_2CONH_2$ | 3,4-Dimethoxy-benzoyl | 215 - 217 |
| $-CH_2CH_2NHCOCH_3$ | 3,4-Dimethoxy-benzoyl | 142 - 143 |
| $-CH_2COOCH_3$ | Nicotinoyl | 117 - 119 |
| 2-(Methoxycarbonyl)-4,5-di-methoxy-phenyl | Nicotinoyl | 121 - 123 |
| $-CH_2COOCH_3$ | $CH_3CO-$ | 114 - 116 |
| $-CH_2COOCH_3$ | 4-Methoxy-benzoyl | 104 - 105 |
| $-CH(CH_3)COOC_2H_5$ | 4-Chloro-benzoyl | 116 - 118 |
| $-CH_2COOCH_3$ | 4-Chloro-benzoyl | 121 - 123 |
| $-CH_2COOCH_3$ | 4-Nitro-benzoyl | 164 - 166 |
| $-CH_2COOCH_3$ | 3-Methoxy-4-acetoxy-benzoyl | 120 - 122 |
| $-CH_2COOCH_3$ | N-Acetyl-2-pyrrolidinyl-carbonyl | 126 - 127 |
| $-CH_2COOCH_3$ | 4-Chlorphenoxy-acetyl | 91 - 92 |

**TABELLE II**

Verbindungen der Formel I mit R = $CH_3$, n = 1 und

| $R^1$ | $R^2$ und $R^3$ zusammen | Fp: (°C) |
|---|---|---|
| 2-Methoxy-carbonyl-4,5,6-trimethoxy-phenyl | Phthaloyl | 146 - 148 |
| $-CH_2COOCH_3$ | Pyridin-2,3-dicarbonyl | 153 - 155 |
| $-CH_2COOCH_3$ | Benzol-1-sulfonyl-2-carbonyl | 126 - 127 |
| 2-(3,4-Dimethoxy-phenyl-ethyl | Phthaloyl | 112 - 113 |

**TABELLE III**

Verbindungen der Formel I mit R = H, $R^1$ = $CH_3$, n = 2 und

| $R^2$ | $R^3$ | Fp: (°C) |
|---|---|---|
| Phthaloyl | | 139 - 141 |
| H | 3,4-Dimethoxy-benzoyl | 117 - 119 |

In den nachfolgenden Beispielen werden pharmazeutische Präparate beschrieben:

**Beispiel 39**

Tabletten können nach folgender Formulierung hergestellt werden:

| | |
|---|---|
| Wirkstoff | 2mg |
| Lactose | 60mg |
| Maisstärke | 30mg |
| lösliche Stärke | 4mg |
| Magnesiumstearat | 4mg |
| | 100mg |

**Beispiel 40**

Für die Herstellung von Weichgelatinekapseln mit 5mg Wirkstoff pro Kapsel eignet sich die folgende Zusammensetzung:

| | |
|---|---|
| Wirkstoff | 5mg |
| Mischung von Triglyzeriden aus Kokosöl | 150mg |
| Kapselinhalt | 155mg |

**Beispiel 41**

Für die Herstellung von Dragees eignet sich folgende Formulierung

| | |
|---|---|
| Wirkstoff | 1mg |
| Maisstärke | 100mg |
| Lactose | 60mg |
| sec Calciumphosphat | 30mg |
| lösliche Stärke | 3mg |
| Magnesiumstearat | 2mg |
| kolloidale Kieselsäure | 4mg |
| | 200mg |

**Beispiel 42**

Injektionslösungen mit 1mg Wirkstoff pro ml können nach folgender Rezeptur hergestellt werden:

| | |
|---|---|
| Wirkstoff | 1,0mg |
| Polyethylenglykol 400 | 0,3mg |
| Natriumchlorid | 2,7mg |
| Wasser zu Injektionszwecken auf | 1 ml |

**Beispiel 43**

Emulsionen mit 3mg Wirkstoff per 5ml können nach folgender Rezeptur hergestellt werden:

| | |
|---|---|
| Wirkstoff | 0,06g |
| Neutralöl | q.s. |
| Natriumcarboxymethylzellulose | 0,6g |
| Polyoxyethylenstearat | q.s. |
| Reinglyzerin | 0,2 bis 2g |
| Aromastoffe | q.s. |
| Wasser (entmineralisiert oder destilliert) ad | 100ml |

**Beispiel 44**

Dragees, enthaltend einen erfindungsgemäßen Wirkstoff und einen anderen therapeutisch wirksamen Stoff:

| | |
|---|---|
| Wirkstoff | 6mg |
| Propranolol | 40mg |
| Milchzucker | 90mg |
| Maisstärke | 90mg |
| sec Calciumphosphat | 34mg |
| lösliche Stärke | 3mg |
| Magnesiumstearat | 3mg |
| kolloidale Kieselsäure | 4mg |
| | 270mg |

**Beispiel 45**

Dragees, enthaltend einen erfindungsgemäßen Wirkstoff und einen anderen therapeutisch wirksamen Stoff:

| | |
|---|---|
| Wirkstoff | 5mg |
| Molsidomin | 5mg |
| Milchzucker | 60mg |
| Maisstärke | 90mg |
| sec Calciumphosphat | 30mg |
| lösliche Stärke | 3mg |
| Magnesiumstearat | 3mg |
| kolloidale Kieselsäure | 4mg |
| | 200mg |

**Beispiel 46**

Kapseln, enthaltend einen erfindungsgemäßen Wirkstoff und einen anderen therapeutisch wirksamen Stoff:

| | |
|---|---|
| Wirkstoff | 5mg |
| Prazosin | 5mg |
| Maisstärke | 185mg |
| | 195mg |

Bei der pharmakologischen Prüfung wurden folgende Ergebnisse erhalten.

1. "Nitrithypoxie"

In diesem Test wird nach der Methode von Gibsen und Bless (J. Neurochem. 27, 1976) bei Mäusen mit $NaNO_2$ (250mg/kg s.c.) eine cerebrale Hypoxie erzeugt, die mit dem Tod der Versuchstiere endet. Bestimmt wird, ob die Überlebenszeit durch Prämedikation mit der Testsubstanz beeinflußt wird. Die Ergebnisse sind in den Tabellen IV bis VII angegeben.

**TABELLE IV**

Prozentuale Verlängerung der Überlebenszeit bei der Verabreichung von 250 mg/kg s.c. $NaNO_2$ und Prämedikation mit Verbindungen der Formel I mit $R^2$ und $R^3$ zusammen Phthaloyl und

| R | $R^1$ | n | Prozentuale Verlängerung |
|---|---|---|---|
| $-CH_3$ | -H | 1 | 17,2 |
| $-C_2H_5$ | $-CH_2COOCH_3$ | 1 | 13,3 |
| $-CH_3$ | $-CH_2COOCH_3$ | 1 | 42 |
| $-CH_3$ | $-CH_2COOC_2H_5$ | 1 | 11 |
| $-CH_3$ | $-CH_2CN$ | 1 | 9 |
| $-CH_3$ | $-CH_2CH_2NHCOCH_3$ | 1 | 30 |
| $-CH_3$ | $-CH_2CO-C(CH_3)_3$ | 1 | 24 |
| $-CH_3$ | $-C_6H_4-2-COO-CH_3$ | 1 | 3,6 |
| $-CH_3$ | $-CH_2CH_2C_6H_3-3,4-(O-CH_3)_2$ | 1 | 2 |
| $-CH_3$ | 2-Methoxycarbonyl-4,5-dimethoxy-phenyl | 1 | 31 |
| $-CH_3$ | 2-(4(5)-Imidazolyl)-ethyl | 1 | 22 |
| $-CH_3$ | $-CH_2CONH_2$ | 1 | 34 |
| -H | $-CH_3$ | 2 | 21 |
| $-C_6H_5$ | $-CH_2COOCH_3$ | 1 | 25 |
| $-CH_3$ | $-CH_2COOCH_3$ | 2 | 15 |
| $-CH_3$ | 2-(Methoxy-carbonyl)-4,5,6-trimethoxy-phenyl | 1 | 14 |
| $-CH_3$ | $-CH_2CH_2N(CH_3)_2$ | 1 | 28 |

**TABELLE V**

Prozentuale Verlängerung der Überlebenszeit bei der Verabreichung von 250 mg/kg s.c. $NaNO_2$ und Prämedikation mit Verbindungen der Formel I mit $R^2$ = H und $R^3$ = 3,4-Dimethoxybenzoyl und

| R | $R^1$ | n | Prozentuale Verlängerung |
|---|---|---|---|
| $-CH_3$ | $-CH_2CH_2NHCOCH_3$ | 1 | 24,2 |
| $-CH_3$ | $-CH_2COOCH_3$ | 1 | 1,6 |
| $-CH_3$ | $-CH_2CONH_2$ | 1 | 28,7 |
| $-CH_3$ | $-CH_2CH_2COOC_2H_5$ | 1 | 4,8 |
| -H | $-CH_3$ | 2 | 12 |
| $-CH_3$ | $-CH_2CH_2CH_2COOC_2H_5$ | 1 | 21,8 |

**TABELLE VI**

Prozentuale Verlängerung der Überlebenszeit bei der Verabreichung von 250 mg/kg s.c. $NaNO_2$ und Prämedikation mit Verbindungen der Formel I mit R = $CH_3$, $R^2$ = H und n = 1 und

| $R^1$ | $R^3$ | Prozentuale Verlängerung |
|---|---|---|
| $-CH_2COOCH_3$ | $4-Cl-C_6H_4-CO-$ | 2 |
| $-CH_2COOCH_3$ | $4-Cl-C_6H_4-OCH_2CO-$ | 2,7 |
| 2-(Methoxy-carbonyl)-4,5-dimethoxy-phenyl | $4-Cl-C_6H_4-OCH_2CO-$ | 34,9 |
| $-CH_2COOCH_3$ | $CH_3CO-$ | 9 |
| $-CH_2COOCH_3$ | $4-NO_2C_6H_4-CO-$ | 7 |
| $-CH_2CH_2NHCOCH_3$ | $4-CH_3O-C_6H_4-CO-$ | 8 |
| $-CH_2COOCH_3$ | $4-CH_3O-C_6H_4-CO-$ | 2 |
| $-CH_2COOCH_3$ | 4-Acetoxy-3-methoxy-benzoyl | 6,4 |
| -H | Nicotinoyl | 16,9 |
| $-CH_2CH_2-C_6H_3-3,4-(OCH_3)_2$ | Nicotinoyl | 12 |
| $-CH(CH_3)COOC_2H_5$ | $4-Cl-C_6H_4-CO-$ | 30 |

**TABELLE VII**

Prozentuale Verlängerung der Überlebenszeit bei der Verabreichung von 250 mg/kg s.c. $NaNO_2$ und Prämedikation mit Verbindungen der Formel I mit R = $CH_3$ und n = 1 und

| $R^1$ | $R^2$ und $R^3$ zusammen | Prozentuale Verlängerung |
|---|---|---|
| $-CH_2COOCH_3$ | Pyridin-2,3-dicarbonyl | 7,8 |
| $-CH_2COOCH_3$ | Phenylen-1-carbonyl-2-sulfenyl | 11,3 |

2. "Passive avoidance""

Die Testapparatur ist eine Hell-Dunkel-Box mit elektrifizierbarem Gitterboden im dunklen Teil.

90 Minuten nach Verabreichung von Kontroll- und Präparatinjektion werden unerfahrene männliche Mäuse mit Scopolaminhydrobromid (3 mg/kg s.c.) behandelt. 5 Minuten später werden die Mäuse in den hellen Teil der Box gesetzt. Nach dem überwechseln in den dunklen Teil der Box erhalten sie einen ihnen unangenehmen elektrischen Fußschock. Nach 24 Stunden wird jede Maus einmal in den hellen Teil der Testapparatur gesetzt und die Verweildauer (max. 180 sec.) gemessen. Die mit einer aktiven Dosis eines Präparats und Scopolamin behandelten Tiere zeigen eine lange Verweildauer, ebenso die nicht mit Scopolamin behandelten Tiere, wogegen diejenigen mit Kontrollinjektion und Scopolamin behandelten eine kurze Verweildauer zeigen.

**TABELLE VIII**

Prozentuale Abschwächung der scopolamininduzierten Amnesie, erkennbar an einer Verlängerung der Zeit bis zum Betreten des dunklen Teils des Passive-Avoidance Testraums. Verbindungen der Formel I mit $R^2$ und $R^3$ zusammen Phthaloyl und

| R | $R^1$ | n | Prozentuale Abschwächung |
|---|---|---|---|
| $-CH_3$ | $-CH_2CONH_2$ | 1 | 67 |
| $-CH_3$ | $-CH_2COOCH_3$ | 1 | 178 |
| $-CH_3$ | $-CH_2COOCH_3$ | 2 | 52 |
| $-CH_3$ | $-H$ | 1 | 10 |
| $-CH_3$ | $-CH_2CH_2CH_2COOC_2H_5$ | 1 | 117 |
| $-CH_3$ | $-CH_2CN$ | 1 | 126 |
| $-CH_3$ | $-CH_2COOC_2H_5$ | 1 | 111 |
| $-CH_3$ | $-CH_2CH_2NHCOCH_3$ | 1 | 167 |
| $-CH_3$ | $-CH_2CH_2N(CH_3)_2$ | 1 | 91 |
| $-CH_3$ | 2-Methoxy-carbonyl-4,5-dimethoxy-phenyl | 1 | 95 |
| $-CH_3$ | $-CH_2CH_2-C_6H_3-3,4-(OCH_3)_2$ | 1 | 115 |
| $-CH_3$ | $-C_6H_4-2-(CO-OCH_3)$ | 1 | 63 |
| $-CH_3$ | $-CH_2CO-C(CH_3)_3$ | 1 | 29 |
| $-C_6H_5$ | $-CH_2COOCH_3$ | 1 | 32 |
| $-C_2H_5$ | $-CH_2COOCH_3$ | 1 | 69 |
| $-H$ | $-CH_3$ | 2 | 59 |

**TABELLE IX**

Prozentuale Abschwächung der scopolamininduzierten Amnesie, erkennbar an einer Verlängerung der Zeit bis zum Betreten des dunklen Teils des Passive-Avoidance Testraums

Verbindungen der Formel I mit $R^2$ = H und $R^3$ = 2,3-Di-methoxybenzoyl und

| R | $R^1$ | n | Prozentuale Abschwächung |
|---|---|---|---|
| $-CH_3$ | $-CH_2CONH_2$ | 1 | 49 |
| $-CH_3$ | $-CH_2CH_2NHCOCH_3$ | 1 | 42 |
| $-CH_3$ | -H | 1 | 87 |
| $-CH_3$ | $-CH_2COOCH_3$ | 1 | 23 |
| $-CH_3$ | $-CH_2CH_2COOC_2H_5$ | 1 | 7 |
| -H | $-CH_3$ | 2 | 64 |

**TABELLE X**

Prozentuale Abschwächung der scopolamininduzierten Amnesie, erkennbar an einer Verlängerung der Zeit bis zum Betreten des dunklen Teils des passive-Avoidance Testraums

Verbindungen der Formel I mit R = $CH_3$, $R^2$ = H und n = 1 und

| $R^1$ | $R^3$ | Prozentuale Abschwächung |
|---|---|---|
| 2-Methoxycarbonyl-4,5-dimethoxy-phenyl | $4-Cl-C_6H_4-OCH_2CO-$ | 91 |
| $-CH_2COOCH_3$ | $CH_3CO-$ | 24 |
| $-CH_2COOCH_3$ | $4-CH_3O-C_6H_4-CO-$ | 20 |
| $-CH_2COOCH_3$ | $4-Cl-C_6H_4-CO-$ | 55 |
| -H | Nicotinoyl | 9 |
| $-CH_2CH_2-C_6H_3-3,4-(OCH_3)_2$ | Nicotinoyl | 62 |

**TABELLE XI**

Prozentuale Abschwächung der scopolamininduzierten Amnesie, erkennbar an einer Verlängerung der Zeit bis zum Betreten des dunklen Teils des Passive-Avoidance Testraums

Verbindungen der Formel I mit R = $CH_3$ und n = 1 und

| $R^1$ | $R^2$ und $R^3$ zusammen | Prozentuale Abschwächung |
|---|---|---|
| $-CH_2COOCH_3$ | Pyridin-2,3-dicarbonyl | 31 |
| $-CH_2COOCH_3$ | Phenylen-1-carbonyl-2-sulfenyl | 35 |

**Patentansprüche**

für die Vertragsstaaten DE, BE, FR, GB, IT, NL, SE, CH

1. 2-(Aminoalkyl)-pyrrol-derivate der allgemeinen Formel

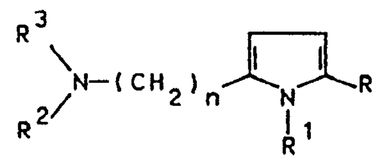

(I)

worin

R: Wasserstoff, Alkyl($C_1$-$C_5$), Phenyl;

$R^1$: Wasserstoff, Alkyl($C_1$-$C_5$), Cyano-alkyl($C_1$-$C_4$), Alkoxy($C_1$-$C_4$)-carbonyl, Alkoxy($C_1C_4$)carbonyl-alkyl($C_1$-$C_4$), $R^7(R^8)$N-carbonyl-alkyl($C_1$-$C_4$), Phenyl, Phenyl-alkyl($C_1$-$C_4$), wobei das Phenyl oder das Phenyl des Phenyl-alkyls auch durch Halogen, Alkoxy($C_1$-$C_4$), Alkyl($C_1$-$C_4$), $R^4(R^5)$N-, Hydroxy, Merkapto, Alkylmerkapto($C_1$-$C_4$), Nitro, Cyano, Alkoxy($C_1$-$C_4$)-carbonyl, Alkoxy($C_1$-$C_4$)-carbonyl-alkyl($C_1$-$C_4$) ein- oder mehrfach substituiert sein kann, Imidazolyl-alkyl($C_1$-$C_4$), $R^4(R^5)N$-$R^6$-, Acetyl-amino-ethyl, Hydroxy-carbonyl-alkyl($C_1$-$C_4$), 1-(Alkoxy($C_1$-$C_4$)-carbo-nyl)-2-mercapto-ethyl, 1-(Alkoxy($C_1$-$C_4$)-carbonyl)-2-hydroxy-ethyl, 1-(Alkoxy($C_1$-$C_4$)-carbonyl)-2-alkoxy-($C_1$-$C_4$)-ethyl, 1-(Alkoxy($C_1$-$C_4$)-carbonyl)-2-alkylthio($C_1$-$C_4$)-ethyl, 1-(Alkoxy($C_1$-$C_4$)-carbonyl)-2-(alkyl($C_1$-$C_4$)-carbonyl-thio)-ethyl, 1-(Alkoxy($C_1$-$C_4$)carbonyl)-2-(alkyl-($C_1$-$C_4$)-carbonyl-oxy)-ethyl, 1-(Alkoxy($C_1$-$C_4$)-carbonyl)-2-imidazolyl-ethyl, 1-(Alkoxy($C_1$-$C_4$)-carbonyl)-2-indolyl-ethyl, 1-(Alkoxy($C_1$-$C_4$)-carbonyl)-2-phenyl-ethyl;

$R^2$, $R^3$: unabhängig voneinander Wasserstoff, Alkyl($C_1$-$C_5$) Alkanoyl($C_1$-$C_5$); durch Amino, Alkoxy($C_1$-$C_4$), Hydroxy, Alkanoyloxy($C_1$-$C_4$), Phenyl, Halogen, ein-, zwei- oder dreifach substituiertes Alkanoyl($C_2$-$C_5$), Benzoyl; durch Halogen, Alkoxy($C_1$-$C_4$), Alkyl($C_1$-$C_4$), Amino, $R^4(R^5)$N-, Hydroxy, Alkanoyloxy($C_1$-$C_4$), Merkapto, Alkyl-merkapto($C_1$-$C_4$), Nitro, Cyano, Alkoxy($C_1$-$C_4$)-carbonyl, Amidosulfonyl ein- oder mehrfach substituiertes Benzoyl, Pyridylcarbonyl, Cycloalkylcarbonyl mit 5 bis 7 C-Atomen im Cycloalkylrest, wobei eine $-CH_2$-Gruppe auch durch -O-, -S- oder $-N(R^{10})$- ersetzt sein kann; (4-Chlorphenoxy)-acetyl; oder $R^2$ und $R^3$ zusammen vicinales Phenylendicarbonyl, Phenylen-1-sulfonyl-2-carbonyl, Alkylen-($C_2$-$C_8$)-dicarbonyl, Alkenylen($C_4$-$C_8$)-dicarbonyl, wobei die Kohlenstoffkette des Alkylen- und Alkenylen-dicarbonyls auch durch -O-, -S-, $-N(R^4)$- ein- oder mehrfach unterbrochen oder ein Spiroatom enthalten kann, über das ein 4-, 5-, 6- oder 7-Ring angebunden sein kann, Phenylen-1-sulfinyl-2-carbonyl, Phenylen-1-sulfenyl-2-carbonyl;

$R^4$ $R^5$: Wasserstoff, Alkyl($C_1$-$C_4$);

$R^6$: Alkylen($C_1$-$C_4$);

$R^7$, $R^8$: unabhängig voneinander Wasserstoff, Alkyl($C_1$-$C_4$) oder zusammen mit dem Stickstoffatom, an das sie gebunden sind, einen 5- oder 6-gliedrigen, gesättigten heterocyclischen Ring bilden, der auch noch -O oder

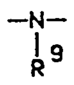

enthalten kann;
$R^9$: Wasserstoff, Alkyl($C_1$-$C_4$), Alkoxy($C_1$-$C_4$), Phenyl, Al-kyl($C_1$-$C_4$)-phenyl, Alkoxy($C_1$-$C_4$)-phenyl;
$R^{10}$: Wasserstoff, Alkanoyl($C_1$-$C_5$);
n: 1, 2 oder 3;
bedeuten, sowie ihre Säureadditionssalze, wobei für die Fälle, in denen $R^2$ und $R^3$ zusammen 1,2-Phenylen-dicarbonyl und gleichzeitig R Methyl oder Ethyl und n die Zahl 1 bedeuten, $R^1$ kein Ethoxy-carbonylmethyl, Ethoxy-carbonyl oder o-Methoxy-carbonylphenyl sein kann und wobei für die Fälle, in denen $R^2$ und $R^3$ zusammen 1,2-Phenylen-dicarbonyl und gleichzeitig R Methyl oder Ethyl und n die Zahl 2 bedeuten, $R^1$ kein Ethoxy-carbonyl oder Ethoxy-carbonylmethyl sein kann und wobei für die Fälle, in denen $R^2$ und $R^3$ zusammen 1,2-Phenylen-dicarbonyl und gleichzeitig n die Zahl 1 und R Phenyl bedeuten, $R^1$ kein o-Methoxy-carbonylphenyl oder Ethoxy-carbonylmethyl sein kann.

2. Verbindungen nach Anspruch 1, dadurch gekennzeichnet, daß R Methyl bedeutet.

3. Verbindungen nach Anspruch 1 und/oder 2, dadurch gekennzeichnet, daß $R^3$ Wasserstoff, Nicotinoyl, 3,4-Dimethoxybenzoyl, 4-Chlorbenzoyl, 4-Chlorphenoxyacetyl, Acetyl bedeutet.

4. Verbindungen nach einem oder mehreren der Ansprüche 1 bis 3, dadurch gekennzeichnet, daß $R^1$ Wasserstoff, 2-Dimethylaminoethyl, 3,4-Dimethoxyphenyl-ethyl, 4,5-Dimeth-oxy-2-(methoxy-carbonyl)-phenyl, Imidazolyl(-4(5))-ethyl, Methoxycarbonyl-methyl, Acetylamino-ethyl, Cyanoethyl, 3-(1-Imidazolyl)-propyl, Aminocarbonylmethyl bedeutet.

5. 1-(Methoxycarbonyl-methyl)-2-(phthalimido-methyl)-5-methyl-pyrrol.

6. 5-Methyl-2-(3,4-dimethoxybenzoyl-amino-methyl)-pyrrol.

7. 1-(2-Methoxy-carbonyl-4,5-dimethoxy-phenyl)-5-methyl-2-(4-chlorophenoxy-acetylamino-methyl)-pyrrol.

8. Verfahren zur Herstellung der Verbindungen der Formel I eines oder mehrerer der Ansprüche 1 bis 7, dadurch gekennzeichnet, daß ein 1,4-Diketon der Formel II
$(R^2)(R^3)N$-$(CH_2)_n$-CO-$CH_2CH_2$-CO-R (II)
oder
ein 1,4-Diketal der Formel IIa

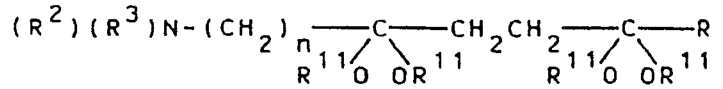

(IIa)

worin $R^{11}$ Alkyl oder zwei benachbarte $R^{11}$ zusammen Alkylen bedeuten, mit einem Amin der Formel III
$H_2NR^1$ (III)
wobei R, $R^1$, $R^2$ und $R^3$ die in den Ansprüchen 1 bis 4 angegebene Bedeutungen besitzen, in einem inerten Lösungsmittel oder Lösungsmittelgemisch bei Temperaturen von 0 bis 200°C umgesetzt wird und die entstandene Verbindung gewünschtenfalls in an sich bekannter Weise in ein Säureadditionssalz überführt wird.

9. Verwendung eines 2-(Aminoalkyl)-pyrrol-derivats der allgemeinen Formel I

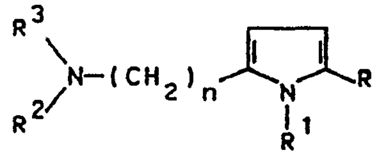

(I)

worin

R: Wasserstoff, Alkyl($C_1$-$C_5$), Phenyl;

$R^1$: Wasserstoff, Alkyl($C_1$-$C_5$), Cyano-alkyl($C_1$-$C_4$), Alk-oxy($C_1$-$C_4$)-carbonyl, Alkoxy($C_1$-$C_4$)carbonyl-alkyl($C_1$-$C_4$), $R^7$($R^8$)N-carbonyl-alkyl($C_1$-$C_4$), Phenyl, Phenyl-alkyl($C_1$-$C_4$), wobei das Phenyl oder das Phenyl des Phenylalkyls auch durch Halogen, Alkoxy($C_1$-$C_4$), Alkyl($C_1$-$C_4$), $R^4$($R^5$)N-, Hydroxy, Merkapto, Alkylmerkapto($C_1$-$C_4$), Nitro, Cyano, Alkoxy($C_1$-$C_4$)-carbonyl, Alkoxy($C_1$-$C_4$)-carbonyl-alkyl($C_1$-$C_4$) ein- oder mehrfach substituiert sein kann, Imidazolyl-alkyl($C_1$-$C_4$), $R^4$($R^5$)N-$R^6$-, Acetyl-amino-ethyl, Hydroxy-carbonyl-alkyl($C_1$-$C_4$), 1-(Alkoxy($C_1$-$C_4$)-carbo-nyl)-2-mercapto-ethyl, 1-(Alkoxy($C_1$-$C_4$)-carbonyl)-2-hydroxy-ethyl, 1-(Alkoxy($C_1$-$C_4$)-carbonyl)-2-alkoxy-($C_1$-$C_4$)-ethyl, 1-(Alkoxy($C_1$-$C_4$)-carbonyl)-2-alkylthio-($C_1$-$C_4$)-ethyl, 1-(Alkoxy($C_1$-$C_4$)-carbonyl)-2-(alkyl($C_1$-$C_4$)-carbonyl-thio)-ethyl, 1-(Alkoxy($C_1$-$C_4$)carbonyl)-2-(alkyl($C_1$-$C_4$)-carbonyl-oxy)-ethyl, 1-(Alkoxy($C_1$-$C_4$)-carbonyl)-2-imidazolyl-ethyl, 1-(Alkoxy($C_1$-$C_4$)-carbonyl)-2-indolyl-ethyl, 1-(Alkoxy($C_1$-$C_4$)-carbonyl)-2-phenyl-ethyl;

$R^2$, $R^3$: unabhängig voneinander Wasserstoff, Alkyl($C_1$-$C_5$), Alkanoyl($C_1$-$C_5$); durch Amino, Alkoxy($C_1$-$C_4$), Hydroxy, Alkanoyloxy($C_1$-$C_4$), Phenyl, Halogen, ein-, zwei- oder dreifach substituiertes Alkanoyl($C_2$-$C_5$), Benzoyl, durch Halogen, Alkoxy($C_1$-$C_4$), Alkyl($C_1$-$C_4$), Amino, $R^4$($R^5$ )N-, Hydroxy, Alkanoyloxy($C_1$-$C_4$), Merkapto, Alkylmerkapto-($C_1$-$C_4$), Nitro, Cyano, Alkoxy($C_1$-$C_4$)-carbonyl, Amidosulfonyl ein- oder mehrfach substituiertes Benzoyl, Pyridylcarbonyl, Cycloalkylcarbonyl mit 5 bis 7 C-Atomen im Cycloalkylrest, wobei eine -$CH_2$-Gruppe auch durch -O-, -S- oder -N($R^{10}$)- ersetzt sein kann; (4-Chlorphenoxy)-acetyl; oder $R^2$ und $R^3$ zusammen vicinales Phenylendicarbonyl, Phenylen-1-sulfonyl-2-carbonyl, Alkylen($C_2$-$C_8$)-di-carbonyl, Alkenylen($C_4$-$C_8$)-dicarbonyl, wobei die Kohlenstoffkette des Alkylen- und Alkenylen-dicarbonyls auch durch -O-, -S-, -N($R^4$)- ein- oder mehrfach unterbrochen oder ein Spiroatom enthalten kann, über das ein 4-, 5-, 6- oder 7-Ring angebunden sein kann, Phenylen-1-sulfinyl-2-carbonyl, Phenylen-1-sulfenyl-2-carbonyl;

$R^4$,$R^5$: Wasserstoff, Alkyl($C_1$-$C_4$);

$R^6$: Alkylen($C_1$-$C_4$);

$R^7$, $R^8$: unabhängig voneinander Wasserstoff, Alkyl($C_1$-$C_4$) oder zusammen mit dem Stickstoffatom, an das sie gebunden sind, einen 5- oder 6-gliedrigen, gesättigten heterocyclischen Ring bilden, der auch noch -O- oder

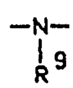

enthalten kann;

$R^9$: Wasserstoff, Alkyl($C_1$-$C_4$), Alkoxy($C_1$-$C_4$), Phenyl, Al-kyl($C_1$-$C_4$)-phenyl, Alkoxy($C_1$-$C_4$)-phenyl,

$R^{10}$: Wasserstoff, Alkanoyl($C_1$-$C_5$) und

n = 1, 2 oder 3

bedeuten oder eines pharmakologisch annehmbaren Säureadditionssalzes zur Herstellung eines Arzneimittels zur Bekämpfung und Vorbeugung von Erkrankungen, die durch eine Einschränkung der Gehirnfunktion bedingt sind, oder zur Behandlung von cerebralen Alterungsvorgängen.

10. Pharmazeutisches Präparat, dadurch gekennzeichnet, daß es als Wirkstoff ein 2-(Aminoalkyl)-pyrrolderivat der allgemeinen Formel I

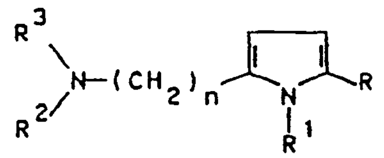

(I)

worin

R: Wasserstoff, Alkyl($C_1$-$C_5$), Phenyl;

$R^1$: Wasserstoff, Alkyl($C_1$-$C_5$), Cyano-alkyl($C_1$-$C_4$), Alk-oxy($C_1$-$C_4$)-carbonyl, Alkoxy($C_1C_4$)carbonyl-alkyl($C_1$-$C_4$), $R^7(R^8)$N-carbonyl-alkyl($C_1$-$C_4$), Phenyl, Phenyl-alkyl($C_1$-$C_4$), wobei das Phenyl oder das Phenyl des Phenyl-alkyls auch durch Halogen, Alkoxy($C_1$-$C_4$), Alkyl($C_1$-$C_4$), $R^4(R^5)$N-, Hydroxy, Merkapto, Alkylmerkapto($C_1$-$C_4$), Nitro, Cyano, Alkoxy($C_1$-$C_4$)-carbonyl, Alkoxy($C_1$-$C_4$)-carbonyl-alkyl($C_1$-$C_4$) ein- oder mehrfach substituiert sein kann, Imidazolyl-alkyl($C_1$-$C_4$), $R^4(R^5)$N-$R^6$-, Acetyl-amino-ethyl, Hydroxy-carbonyl-alkyl($C_1$-$C_4$), 1-(Alkoxy($C_1$-$C_4$)-carbo-nyl)-2-mercapto-ethyl, 1-(Alkoxy($C_1$-$C_4$)-carbonyl)-2-hydroxy-ethyl, 1-(Alkoxy($C_1$-$C_4$)-carbonyl)-2-alkoxy-($C_1$-$C_4$)-ethyl, 1-(Alkoxy($C_1$-$C_4$)-carbonyl)-2-alkylthio-($C_1$-$C_4$)-ethyl, 1-(Alkoxy($C_1$-$C_4$)-carbonyl)-2-(alkyl($C_1$-$C_4$)-carbonyl-thio)-ethyl, 1-(Alkoxy($C_1$-$C_4$)carbonyl)-2-(alkyl($C_1$-$C_4$)-carbonyl-oxy)-ethyl, 1-(Alkoxy($C_1$-$C_4$)-car-bonyl)-2-imidazolyl-ethyl, 1-(Alkoxy($C_1$-$C_4$)-carbonyl)-2-indolyl-ethyl, 1-(Alkoxy($C_1$-$C_4$)-carbonyl)-2-phenyl-ethyl;

$R^2$, $R^3$ : unabhängig voneinander Wasserstoff, Alkyl($C_1$-$C_5$), Alkanoyl($C_1$-$C_5$); durch Amino, Alkoxy($C_1$-$C_4$), Hydroxy, Alkanoyloxy($C_1$-$C_4$), Phenyl, Halogen, ein-, zwei- oder dreifach substituiertes Alkanoyl($C_2$-$C_5$, Benzoyl, durch Halogen, Alkoxy($C_1$-$C_4$), Alkyl($C_1$-$C_4$), Amino, $R^4(R^5)$N-, Hydroxy, Alkanoyloxy($C_1$-$C_4$), Merkapto, Alkylmerkapto-($C_1$-$C_4$), Nitro, Cyano, Alkoxy($C_1$-$C_4$)-carbonyl, Amidosulfonyl ein- oder mehrfach substituiertes Benzoyl, Pyridylcarbonyl, Cycloalkylcarbonyl mit 5 bis 7 C-Atomen im Cycloalkylrest, wobei eine -$CH_2$-Gruppe auch durch -O-, -S- oder -N($R^{10}$)- ersetzt sein kann; (4-Chlorphenoxy)-acetyl; oder $R^2$ und $R^3$ zusammen vicinales Phenylendicarbonyl, Phenylen-1-sulfonyl-2-carbonyl, Alkylen($C_2$-$C_8$) dicarbonyl, Alkenylen($C_4$-$C_8$)-dicarbonyl, wobei die Kohlenstoffkette des Alkylen- und Alkenylen-dicarbonyls auch durch -O-, -S-, -N($R^4$)- ein- oder mehrfach unterbrochen oder ein Spiroatom enthalten kann, über das ein 4-, 5-, 6- oder 7-Ring angebunden sein kann, Phenylen-1-sulfinyl-2-carbonyl, Phenylen-1-sulfenyl-2-carbonyl;

$R^4$,$R^5$: Wasserstoff, Alkyl($C_1$-$C_4$);

$R^6$: Alkylen($C_1$-$C_4$);

$R^7$, $R^8$: unabhängig voneinander Wasserstoff, Alkyl($C_1$-$C_4$) oder zusammen mit dem Stickstoffatom, an das sie gebunden sind, einen 5- oder 6-gliedrigen, gesättigten heterocyclischen Ring bilden, der auch noch -O-oder

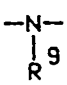

enthalten kann;

$R^9$ : Wasserstoff, Alkyl($C_1$-$C_4$), Alkoxy($C_1$-$C_4$), Phenyl, Al-kyl($C_1$-$C_4$)-phenyl, Alkoxy($C_1$-$C_4$)-phenyl,

$R^{10}$: Wasserstoff, Alkanoyl($C_1$-$C_5$) und

n = 1, 2 oder 3

bedeuten oder ein pharmazeutisch annehmbares Säureadditionssalz davon, zusammen mit einem pharmazeutisch annehmbaren Trägerstoff und gegebenenfalls pharmazeutisch annehmbaren Zusatzstoffen und gegebenenfalls noch einen oder mehrere andere pharmakologische Wirkstoffe enthält.

**Patentansprüche**

für den Vertragsstaat AT

1. Verfahren zur Herstellung von 2-(Aminoalkyl)-pyrrol-derivate der allgemeinen Formel I

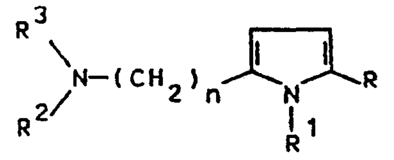 (I)

worin

R: Wasserstoff, Alkyl($C_1$-$C_5$), Phenyl;,

$R^1$: Wasserstoff, Alkyl($C_1$-$C_5$), Cyano-alkyl($C_1$-$C_4$), Alk-oxy($C_1$-$C_4$)-carbonyl, Alkoxy($C_1$-$C_4$)carbonyl-alkyl($C_1$-$C_4$), $R^7$($R^8$)N-carbonyl-alkyl($C_1$-$C_4$), Phenyl, Phenyl-alkyl($C_1$-$C_4$), wobei das Phenyl oder das Phenyl des Phenyl-alkyls auch durch Halogen, Alkoxy($C_1$-$C_4$), Alkyl($C_1$-$C_4$), $R^4$($R^5$)M-, Hydroxy, Merkapto, Alkylmerkapto($C_1$-$C_4$), Nitro, Cyano, Alkoxy($C_1$-$C_4$)-carbonyl, Alkoxy($C_1$-$C_4$)-carbonyl-alkyl($C_1$-$C_4$) ein- oder mehrfach substituiert sein kann, Imidazolyl-alkyl($C_1$-$C_4$), $R^4$($R^5$)N-$R^6$-, Acetyl-amino-ethyl, Hydroxy-carbonyl-alkyl($C_1$-$C_4$), 1-(Alkoxy($C_1$-$C_4$)-carbonyl)-2-mercapto-ethyl, 1-(Alkoxy($C_1$-$C_4$)-carbonyl)-2-hydroxy-ethyl, 1-(Alkoxy($C_1$-$C_4$)-carbonyl)-2-alkoxy-($C_1$-$C_4$)-ethyl, 1-(Alkoxy($C_1$-$C_4$)-carbonyl)-2-alkylthio-($C_1$-$C_4$)-ethyl, 1-(Alkoxy($C_1$-$C_4$)-carbonyl)-2-(alkyl($C_1$-$C_4$)-carbonyl-thio)-ethyl, 1-(Alkoxy($C_1$-$C_4$)carbonyl)-2-(alkyl($C_1$-$C_4$)-carbonyl-oxy)-ethyl, 1-(Alkoxy($C_1$-$C_4$)-car-bonyl)-2-imidazolyl-ethyl, 1-(Alkoxy($C_1$-$C_4$)-carbonyl)-2-indolyl-ethyl, 1-(Alkoxy($C_1$-$C_4$)-carbonyl)-2-phenyl-ethyl;,

$R^2$, $R^3$: unabhängig voneinander Wasserstoff, Alkyl($C_1$-$C_5$), Alkanoyl($C_1$-$C_5$); durch Amino, Alkoxy($C_1$-$C_4$), Hydroxy, Alkanoyloxy($C_1$-$C_4$), Phenyl, Halogen, ein-, zwei- oder dreifach substituiertes Alkanoyl($C_2$-$C_5$), Benzoyl, durch Halogen, Alkoxy($C_1$-$C_4$), Alkyl($C_1$-$C_4$), Amino, $R^4$($R^5$)N-, Hydroxy, Alkanoyloxy($C_1$-$C_4$), Merkapto, Alkyl-merkapto($C_1$-$C_4$), Nitro, Cyano, Alkoxy($C_1$-$C_4$)-carbonyl, Amidosulfonyl ein- oder mehrfach substituiertes Benzoyl, Pyridylcarbonyl, Cycloalkylcarbonyl mit 5 bis 7 C-Atomen im Cycloalkylrest, wobei eine -$CH_2$-Gruppe auch durch -O-, -S- oder -N($R^{10}$)- ersetzt sein kann; (4-Chlorphenoxy)-acetyl; oder $R^2$ und $R^3$ zusammen vicinales Phenylendicarbonyl, Phenylen-1-sulfonyl-2-carbonyl, Alkylen($C_2$-$C_8$)-dicarbonyl, Alkenylen($C_4$-$C_8$)-dicarbonyl, wobei die Kohlenstoffkette des Alkylen- und Alkenylen-dicarbonyls auch durch -O-, -S-, -N($R^4$)- ein- oder mehrfach unterbrochen oder ein Spiroatom enthalten kann, über das ein 4-, 5-, 6- oder 7-Ring angebunden sein kann, Phenylen-1-sulfinyl-2-carbonyl, Phenylen-1-sulfenyl-2-carbonyl;

$R^4$,$R^5$: Wasserstoff, Alkyl($C_1$-$C_4$);

$R^6$: Alkylen($C_1$-$C_4$);

$R^7$, $R^8$: unabhängig voneinander Wasserstoff, Alkyl($C_1$-$C_4$) oder zusammen mit dem Stickstoffatom, an das sie gebunden sind, einen 5- oder 6-gliedrigen, gesättigten heterocyclischen Ring bilden, der auch noch -O- oder

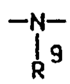

enthalten kann;

$R^9$: Wasserstoff, Alkyl($C_1$-$C_4$), Alkoxy($C_1$-$C_4$), Phenyl, Al-kyl($C_1$-$C_4$)-phenyl, Alkoxy($C_1$-$C_4$)-phenyl;

$R^{10}$: Wasserstoff, Alkanoyl($C_1$-$C_5$);

n: 1, 2 oder 3;

bedeuten, sowie ihrer Säureadditionssalze, wobei für die Fälle, in denen $R^2$ und $R^3$ zusammen 1,2-Phenylen-dicarbonyl und gleichzeitig R Methyl oder Ethyl und n die Zahl 1 bedeuten, $R^1$ kein Ethoxy-carbonylmethyl, Ethoxy-carbonyl oder o-Methoxy-carbonylphenyl sein kann und wobei für die Fälle, in denen $R^2$ und $R^3$ zusammen 1,2-Phenylen-dicarbonyl und gleichzeitig R Methyl oder Ethyl und n die Zahl 2 bedeuten, $R^1$ kein Ethoxy-carbonyl oder Ethoxy-carbonylmethyl sein kann und wobei für die Fälle, in denen $R^2$ und $R^3$ zusammen 1,2-Phe-nylen-dicarbonyl und gleichzeitig n die Zahl 1 und R Phenyl bedeuten, $R^1$ kein o-Methoxy-carbonylphenyl oder Ethoxy-carbonylmethyl sein kann, dadurch gekennzeichnet, daß ein 1,4-Diketon der Formel II

$(R^2)(R^3)N\text{-}(CH_2)_n\text{-}CO\text{-}CH_2CH_2\text{-}CO\text{-}R$ (II)

oder

ein 1,4-Diketal der Formel IIa

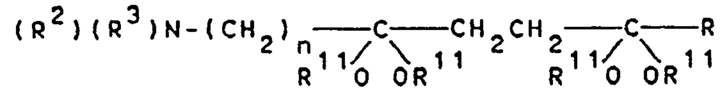 (IIa)

worin $R^{11}$ Alkyl oder zwei benachbarte $R^{11}$ zusammen Alkylen bedeuten, mit einem Amin der Formel III

$H_2NR^1$ (III)

in einem inerten Lösungsmittel oder Lösungsmittelgemisch bei Temperaturen von 0 bis 200°C umgesetzt wird und die entstandene Verbindung gewünschtenfalls in an sich bekannter Weise in ein Säureadditionssalz überführt wird.

2. Verfahren nach Anspruch 1, dadurch gekennzeichnet, daß die Ausgangsverbindungen so ausgewählt werden, daß Verbindungen der Formel I oder Säureadditionssalze davon entstehen, wobei R Methyl bedeutet.

3. Verfahren nach Anspruch 1 und/oder 2, dadurch gekennzeichnet, daß die Ausgangsverbindungen so ausgewählt werden, daß Verbindungen der Formel I oder Säureadditionssalze davon entstehen, wobei $R^3$ Wasserstoff, Nicotinoyl, 3,4-Dimethoxybenzoyl, 4-Chlorbenzoyl, 4-Chlor-phenoxyacetyl, Acetyl bedeutet.

4. Verfahren nach einem oder mehreren der Ansprüche 1 bis 3, dadurch gekennzeichnet, daß die Ausgangsverbindungen so ausgewählt werden, daß Verbindungen der Formel I oder Säureadditionssalze davon entstehen, wobei $R^1$ Wasserstoff, 2-Dimethylaminoethyl, 3,4-Dimethoxyphenyl-ethyl, 4,5-Dimethoxy-2-(methoxy-carbonyl)-phenyl, Imidazolyl-(-4(5))-ethyl, Methoxycarbonyl-methyl, Acetylamino-ethyl, Cyanoethyl, 3-(1-Imidazolyl)-propyl, Aminocarbonylmethyl bedeutet.

5. Verfahren nach einem oder mehreren der Ansprüche 1 bis 4, dadurch gekennzeichnet, daß die Ausgangsverbindungen so ausgewählt werden, daß die Verbindung 1-(Methoxycar-bonyl-methyl)-2-(phthalimido-methyl)-5-methyl-pyrrol entsteht.

6. Verfahren nach einem oder mehreren der Ansprüche 1 bis 4, dadurch gekennzeichnet, daß die Ausgangsverbindungen so ausgewählt werden, daß die Verbindung 5-Methyl-2-(3,4-dimethoxybenzoyl-amino-methyl)-pyrrol entsteht.

7. Verfahren nach einem oder mehreren der Ansprüche 1 bis 4, dadurch gekennzeichnet, daß die Ausgangsverbindungen so ausgewählt werden, daß die Verbindung 1-(2-Methoxy-carbonyl-4,5-dimethoxy-phenyl)-5-methyl-2-(4chlorophen-oxy-acetylamino-methyl)-pyrrol entsteht.

8. Verfahren zur Herstellung von pharmazeutischen Präparaten, die nootrope Wirkstoffe enthalten und die zur Behandlung von Menschen zur Bekämpfung und Vorbeugung von Erkrankungen, die durch eine Einschränkung der Gehirnfunktion bedingt sind oder zur Behandlung von cerebralen Alterungsvorgängen verwendet werden, dadurch gekennzeichnet, daß ein 2-(Aminoalkyl)-pyrrol-derivat der allgemeinen Formel I

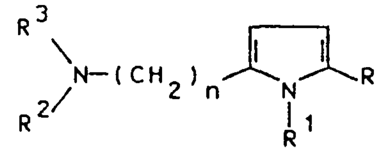 (I)

worin

R: Wasserstoff, Alkyl($C_1$-$C_5$), Phenyl;

$R^1$: Wasserstoff, Alkyl($C_1$-$C_5$), Cyano-alkyl($C_1$-$C_4$), Alkoxy($C_1$-$C_4$)-carbonyl, Alkoxy($C_1$-$C_4$)carbonyl-alkyl($C_1$-$C_4$), $R^7$($R^8$)N-carbonyl-alkyl($C_1$-$C_4$), Phenyl, Phenyl-alkyl($C_1$-$C_4$), wobei das Phenyl oder das Phenyl des Phenyl-alkyls auch durch Halogen, Alkoxy($C_1$-$C_4$), Alkyl($C_1$-$C_4$), $R^4$($R^5$)N-, Hydroxy, Merkapto, Alkylmerkapto($C_1$-$C_4$), Nitro, Cyano, Alkoxy($C_1$-$C_4$)-carbonyl, Alkoxy($C_1$-$C_4$)-carbonyl-alkyl($C_1$-$C_4$) ein- oder mehrfach substituiert sein kann, Imidazolyl-alkyl($C_1$-$C_4$), $R^4$($R^5$)N-$R^6$-, Acetyl-amino-ethyl, Hydroxy-carbonyl-alkyl($C_1$-$C_4$), 1-(Alkoxy($C_1$-$C_4$)-carbonyl)-2-mercapto-ethyl, 1-(Alkoxy($C_1$-$C_4$)-carbonyl)-2-hydroxy-ethyl, 1-(Alkoxy($C_1$-$C_4$)-carbonyl)-2-alkoxy-($C_1$-$C_4$)-ethyl, 1-(Alkoxy($C_1$-$C_4$)-carbonyl)-2-alkylthio-($C_1$-$C_4$)-ethyl, 1-(Alkoxy($C_1$-$C_4$)-carbonyl)-2-(alkyl($C_1$-$C_4$)-carbonyl-thio)-ethyl, 1-(Alkoxy($C_1$-$C_4$)carbonyl)-2-(alkyl($C_1$-$C_4$)-carbonyl-oxy)-ethyl, 1-(Alkoxy($C_1$-$C_4$)-car-bonyl)-2-imidazolyl-ethyl, 1-(Alkoxy($C_1$-$C_4$)-carbonyl)-2-indolyl-ethyl, 1-(Alkoxy($C_1$-$C_4$)-carbonyl)-2-phenyl-ethyl;

$R^2$ $R^3$: unabhängig voneinander Wasserstoff Alkyl($C_1$-$C_5$) Alkanoyl($C_1$-$C_5$); durch Amino, Alkoxy($C_1$-$C_4$), Hydroxy, Alkanoyloxy($C_1$-$C_4$), Phenyl, Halogen, ein-, zwei- oder dreifach substituiertes Alkanoyl($C_2$-$C_5$),

Benzoyl, durch Halogen, Alkoxy($C_1$-$C_4$), Alkyl($C_1$-$C_4$), Amino, $R^4(R^5)N$-, Hydroxy, Alkanoyloxy($C_1$-$C_4$), Merkapto, Alkylmerkapto-($C_1$-$C_4$), Nitro, Cyano, Alkoxy($C_1$-$C_4$)-carbonyl, Amidosulfonyl ein- oder mehrfach substituiertes Benzoyl, Pyridylcarbonyl, Cycloalkylcarbonyl mit 5 bis 7 C-Atomen im Cycloalkylrest, wobei eine $-CH_2$-Gruppe auch durch -O-, -S- oder $-N(R^{10})$- ersetzt sein kann; (4-Chlor-phenoxy)-acetyl; oder $R^2$ und $R^3$ zusammen vicinales Phenylendicarbonyl, Phenylen-1-sulfonyl-2-carbonyl, Alkylen-($C_2$-$C_8$)-dicarbonyl, Alkenylen($C_4$-$C_8$)-dicarbonyl, wobei die Kohlenstoffkette des Alkylen- und Alkenylen-dicarbonyls auch durch -O-, -S-, $-N(R^4)$- ein- oder mehrfach unterbrochen oder ein Spiroatom enthalten kann, über das ein 4-, 5-, 6- oder 7-Ring angebunden sein kann, Pheny-len-1-sulfinyl-2-carbonyl, Phenylen-1-sulfenyl-2-carbonyl;

$R^4$,$R^5$: Wasserstoff, Alkyl($C_1$-$C_4$);

$R^6$: Alkylen($C_1$-$C_4$);

$R^7$, $R^8$: unabhängig voneinander Wasserstoff, Alkyl($C_1$-$C_4$) oder zusammen mit dem Stickstoffatom, an das sie gebunden sind, einen 5- oder 6-gliedrigen, gesättigten heterocyclischen Ring bilden, der auch noch -O- oder

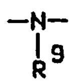

enthalten kann;

$R^9$: Wasserstoff, Alkyl($C_1$-$C_4$), Alkoxy($C_1$-$C_4$), Phenyl, Al-kyl($C_1$-$C_4$)-phenyl, Alkoxy($C_1$-$C_4$)-phenyl,

$R^{10}$: Wasserstoff, Alkanoyl($C_1$-$C_5$) und

n = 1, 2 oder 3

bedeuten oder ein pharmakologisch annehmbares Säureadditionssalz davon als Wirkstoff dadurch hergestellt wird, daß ein 1,4-Diketon der Formel II

$(R^2)(R^3)N-(CH_2)_n-CO-CH_2CH_2-CO-R$ (II)

oder

ein 1,4-Diketal der Formel IIa

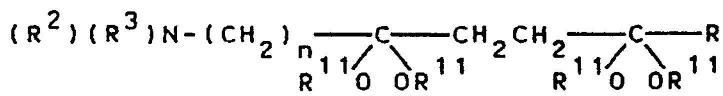

(IIa)

worin $R^{11}$ Alkyl oder zwei benachbarte $R^{11}$ zusammen Alkylen bedeuten, mit einem Amin der Formel III

$H_2NR^1$

in einem inerten Lösungsmittel oder Lösungsmittelgemisch bei Temperaturen von 0 bis 200°C umgesetzt und gegebenenfalls in ein Säureadditionssalz überführt wird und daß die Verbindung der Formel I oder ihr Säureadditionssalz isoliert und der so erhaltene Wirkstoff in an sich bekannter Weise durch Mischen mit einem oder mehreren pharmazeutisch annehmbaren Trägerstoffen in ein verabreichungsfähiges pharmazeutisches Präparat gebracht wird.

## Claims

for the Contracting States: DE, BE, FR, GB, IT, NL, SE, CH

1. 2-(Aminoalkyl)-pyrrole derivatives of the general formula I

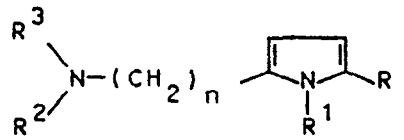 (I)

wherein R denotes hydrogen, alkyl($C_1$-$C_5$) or phenyl; $R^1$ denotes hydrogen, alkyl($C_1$-$C_5$), cyanü-alkyl($C_1$-$C_4$), alkoxy($C_1$-$C_4$)-carbonyl, alkoxy($C_1$-$C_4$)-carbonyl-alkyl-($C_1$-$C_4$), $R^7$($R^8$)N-carbonyl-alkyl($C_1$-$C_4$), phenyl or phenyl-alkyl($C_1$-$C_4$), it being possible for the phenyl or the phenyl of the phenyl-alkyl also to be monosubstituted or polysubstituted by halogen, alkoxy($C_1$-$C_4$), alkyl-($C_1$-$C_4$), $R^4$($R^5$)N-, hydroxyl, mercapto, alkylmercapto-($C_1$-$C_4$), nitro, cyano, alkoxy($C_1$-$C_4$)-carbonyl or alkoxy-($C_1$-$C_4$)-carbonyl-alkyl($C_1$-$C_4$), or R denotes imidazolyl-alkyl($C_1$-$C_4$), $R^4$($R^5$)N-$R^6$-, acetyl-amino-ethyl, hydroxy-carbonyl-alkyl($C_1$-$C_4$), 1-(alkoxy($C_1$-$C_4$)carbonyl)-2-mercapto-ethyl, 1-(alkoxy($C_1$-$C_4$)-carbonyl)-2-hydroxy-ethyl, 1-(alkoxy($C_1$-$C_4$)-carbonyl)-2-alkoxy($C_1$-$C_4$)-ethyl, 1-(alkoxy($C_1$-$C_4$)-carbonyl)-2-alkylthio($C_1$-$C_4$)-ethyl, 1-(alkoxy($C_1$-$C_4$)-carbonyl)-2-(alkyl($C_1$-$C_4$)-carbonyl-thio)-ethyl, 1-(alkoxy($C_1$-$C_4$)-carbonyl)-2-(alkyl($C_1$-$C_4$)-carbonyl-oxy)-ethyl, 1-(alkoxy($C_i$-$C_4$)-carbonyl-2-imidazolyl-ethyl, 1-(alkoxy($C_1$-$C_4$)-carbonyl)-2-indolyl-ethyl or 1-(alkoxy($C_1$-$C_4$)-carbonyl)-2-phenyl-ethyl; $R^2$ and $R^3$ independently of one another denote hydrogen, alkyl($C_1$-$C_5$), alkanoyl($C_1$-$C_5$), alkanoyl($C_2$-$C_5$) which is mono-, di- or trisubstituted by amino, alkoxy($C_1$-$C_4$), hydroxyl, alkanoyl-oxy($C_1$-$C_4$), phenyl or halogen, benzoyl, benzoyl which is mono- or polysubstituted by halogen, alkoxy($C_1$-$C_4$), alkyl($C_1$-$C_4$), amino, $R^4$($R^5$)N-, hydroxy alkanoyloxy($C_1$-$C_4$), mercapto, alkylmércap-to ($C_1$-$C_4$) nitro cyano, alkoxy($C_1$-$C_4$)-carbonyl or amidosulphonyl, or pyridylcarbonyl, cycloalkylcarbonyl having 5 to 7 C atoms in the cycloalkyl radical, it being possible for one -$CH_2$-group to be replaced by -O-, -S- or -N($R^{10}$)-; (4-chlorophenoxy)-acetyl; or $R^2$ and $R^3$ together denote vicinal phenylenedicarbonyl, phenylene-1-sulphonyl-2-carbonyl, alkylene($C_2$-$C_8$)-dicarbonyl or alkenylene($C_4$-$C_8$)-dicarbonyl, is being possible for the carbon chain of the alkylene- and alkenylene-dicarbonyl also to be interrupted by one or more -O-, -S- or -N($R^4$)-groups, or to contain a spiro-atom, via which a 4-, 5-, 6- or 7-membered ring may be bonded; or phenylene-1-sulphinyl-2-carbonyl or phenylene-1-sulphenyl-2-carbonyl; $R^4$ and $R^5$ denote hydrogen or alkyl($C_1$-$C_4$); $R^6$ denotes alkylene($C_1$-$C_4$); $R^7$ and $R^8$ independently of one another denote hydrogen or alkyl($C_1$-$C_4$), or, together with the nitrogen atom to which they are bonded, form a 5- or 6-membered, saturated heterocyclic ring, which may also additionally contain -O- or

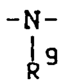 ;

$R^9$ denotes hydrogen, alkyl($C_1$-$C_4$), alkoxy($C_1$-$C_4$), phenyl, alkyl($C_1$-$C_4$)-phenyl or alkoxy($C_1$-$C_4$)-phenyl; $R^{10}$ denotes hydrogen or alkanoyl($C_1$-$C_5$); and n denotes 1, 2 or 3; and their acid addition salts, and in the cases where $R^2$ and $R^3$ together denote 1,2-phenylene-dicarbonyl and at the same time R denotes methyl or ethyl and n denotes the number 1, $R^1$ cannot be ethoxy-carbonyl-methyl, ethoxy-carbonyl or o-methoxy-carbonylphenyl, and, in the cases where $R^2$ and $R^3$ together denote 1,2-phenylene-dicarbonyl and at the same time R denotes methyl or ethyl and n denotes the number 2, $R^1$ cannot be ethoxy-carbonyl or ethoxycarbonylmethyl, and, in the cases where $R^2$ and $R^3$ together denote 1,2-phenylene-dicarbonyl and at the same time n denotes the number 1 and R denotes phenyl, $R^1$ cannot be o-methoxy-carbonylphenyl or ethoxy-carbonylmethyl.

2. Compounds according to Claim 1, characterised in that R denotes methyl.

3. Compounds according to Claims 1 and/or 2, characterised in that $R^3$ denotes hydrogen, nicotinoyl, 3,4-dimethoxybenzoyl, 4-chlorobenzoyl, 4-chlorophenoxy-acetyl or acetyl.

4. Compounds according to one or several of Claims 1 to 3, characterised in that $R^1$ denotes hydrogen, 2-dimethylaminoethyl, 3,4-dimethoxyphenyl-ethyl, 4,5-dimethoxy-2-(methoxycarbonyl)-phenyl, imidazol-4- or -5-yl-ethyl, methoxycarbonylmethyl, acetylamino-ethyl, cyanoethyl, 3-(1-imidazolyl)-propyl or aminocarbonylmethyl.

5. 1-(Methoxycarbonyl-methyl)-2-(phthalimido-methyl)-5-methyl-pyrrole.

6. 5-Methyl-2-(3,4-dimethoxybenzoyl-amino-methyl)-pyrrole.

7. 1-(2-Methoxy-carbonyl-4,5-dimethoxy-phenyl)-5-methyl-2-(4-chlorophenoxy-acetylamino-methyl)-pyrrole.

8. Process for the preparation of the compounds of the formula I of one or several of Claims 1 to 7, characterised in that a 1,4-diketone of the formula II

$(R^2)(R^3)N-(CH_2)_n-CO-CH_2CH_2-CO-R$ (II)
or a 1,4-diketal of the formula IIa

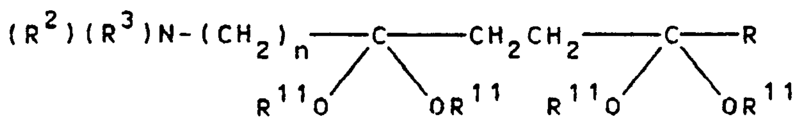
(IIa)

wherein $R^{11}$ denotes alkyl or two adjacent radicals $R^{11}$ together denote alkylene, is reacted with an amine of the formula III

$H_2NR^1$

wherein R, $R^1$, $R^2$ and $R^3$ have the meanings indicated in Claims 1 to 4, in an inert solvent or solvent mixture at temperatures of 0 to 200°C and the compound is converted, if desired, into an acid addition salt in a manner known by itself.

9. Use of a 2-(amino-alkyl)-pyrrole derivative of the general formula I

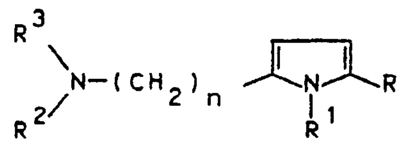
(I)

wherein R denotes hydrogen, alkyl($C_1$-$C_5$) or phenyl; $R^1$ denotes hydrogen, alkyl($C_1$-$C_5$), cyano-alkyl($C_1$-$C_4$), alkoxy($C_1$-$C_4$)-carbonyl, alkoxy($C_1$-$C_4$)-carbonyl-alkyl-($C_1$-$C_4$), $R^7(R^8)$N-carbonyl-alkyl($C_1$-$C_4$), phenyl or phenyl-alkyl($C_1$-$C_4$), it being possible for the phenyl or the phenyl of the phenyl-alkyl also to be monosubstituted or polysubstituted by halogen, alkoxy($C_1$-$C_4$), alkyl-($C_1$-$C_4$), $R^4(R^5)$N-, hydroxyl, mercapto, alkylmercapto-($C_1$-$C_4$), nitro, cyano, alkoxy($C_1$-$C_4$)-carbonyl or alkoxy-($C_1$-$C_4$)-carbonyl-alkyl($C_1$-$C_4$), or R denotes imidazolyl-alkyl($C_1$-$C_4$), $R^4(R^5)N-R^6$-, acetyl-amino-ethyl, hydroxy-carbonyl-alkyl($C_1$-$C_4$), 1-(alkoxy($C_1$-$C_4$)-carbonyl-2-mercapto-ethyl, 1-(alkoxy($C_1$-$C_4$)-carbonyl)-2-hydroxy-ethyl, 1-(alkoxy($C_1$-$C_4$)-carbonyl)-2-alkoxy($C_1$-$C_4$)-ethyl, 1-(alkoxy($C_1$-$C_4$)-carbonyl)-2-alkylthio($C_1$-$C_4$)-ethyl, 1-(alkoxy($C_1$-$C_4$)-carbonyl)-2-(alkyl($C_1$-$C_4$)-carbonyl-thio)-ethyl, 1-(alkoxy($C_1$-$C_4$)-carbonyl)-2-(alkyl($C_1$-$C_4$)-carbonyl-oxy)-ethyl, 1-(alkoxy($C_1$-$C_4$)-carbonyl)-2-imidazolyl-ethyl, 1-(alkoxy($C_1$-$C_4$)-carbonyl-2-indolyl-ethyl or 1-(alkoxy($C_1$-$C_4$)-carbonyl)-2-phenyl-ethyl; $R^2$ and $R^3$ independently of one another denote hydrogen, alkyl($C_1$-$C_5$) alkanoyl($C_1$-$C_5$), alkanoyl($C_2$-$C_5$), which is mono-, di- or trisubstituted by amino, alkoxy($C_1$-$C_4$), hydroxyl, alkanoyloxy($C_1$-$C_4$), phenyl or halogen, benzoyl, benzoyl which is mono- or polysubstituted by halogen, alkoxy($C_1$-$C_4$) alkyl($C_1$-$C_4$), amino $R^4(R^5$N-, hydroxyl alkanoyloxy($C_1$-$C_4$), mercapto, alkylmercapto($C_1$-$C_4$), nitro, cyano, alkoxy($C_1$-$C_4$)-carbonyl- or amidosulphonyl, or pyridylcarbonyl, cycloalkylcarbonyl having 5 to 7 C atoms in the cycloalkyl radical, it being possible for one $-CH_2$-group to be replaced by -O-, -S-, or $-N(R^{10})$-; (4-Chlorophenoxy)-acetyl; or $R^2$ and $R^3$ together denote vicinal phenylene-dicarbonyl, phenylene-1-sulphonyl-2-carbonyl, alkylene($C_2$-$C_8$)-dicarbonyl or alkenylene-($C_4$-$C_8$)-dicarbonyl, it being possible for the carbon . chain of the alkylene- and alkenylene-dicarbonyl also to be interrupted by one or more -O-, -S- or $-N(R^4)$-groups, or to contain a spiro atom, via which a 4-, 5-, 6- or 7-membered ring may be bonded; or phenylene-1-sulphinyl-2-carbonyl or phenylene-1-sulphenyl-2-carbonyl; $R^4$ and $R^5$ denote hydrogen or alkyl($C_1$-$C_4$); $R^6$ denotes alkylene-($C_1$-$C_4$); $R^7$ and R independently of one another denote hydrogen or alkyl($C_1$-$C_4$), or, together with the nitrogen atom to which they are bonded, form a 5- or 6-membered, saturated heterocyclic ring, which may also additionally contain -O- or

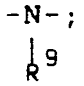

$R^9$ denotes hydrogen, alkyl($C_1$-$C_4$), alkoxy($C_1$-$C_4$), phenyl, alkyl($C_1$-$C_4$) or or alkoxy($C_1$-$C_4$)-phenyl; $R^{10}$ denotes hydrogen or alkanoyl($C_1$-$C_5$); and n denotes 1,2 or 3, or a pharmacologically acceptable acid addition salt for preparing a medicament for combating and preventing disorders which are caused by a restriction in cerebral function, or for the treatment of cerebral aging processes.

10. Pharmaceutical product characterised in that it contains, as the active compound, a 2-(aminoalkyl)-pyrrole derivative of the general formula I

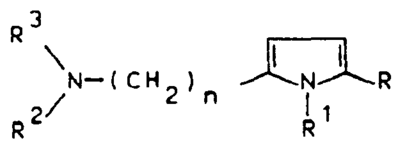 (I)

wherein R denotes hydrogen, alkyl($C_1$-$C_5$) or phenyl; $R^1$ denotes hydrogen, alkyl($C_1$-$C_5$), cyano-alkyl($C_1$-$C_4$), alkoxy($C_1$-$C_4$)-carbonyl, alkoxy($C_1$-$C_4$)-carbonyl-alkyl-($C_1$-$C_4$, $R^7$($R^8$)N-carbonyl-alkyl($C_1$-$C_4$), phenyl or phenyl-alkyl($C_1$-$C_4$), it being possible for the phenyl or the phenyl of the phenyl-alkyl also to be monosubstituted or polysubstituted by halogen, alkoxy($C_1$-$C_4$), alkyl($C_1$-$C_4$), $R^4$($R^5$N-, hydroxyl, mercapto, alkylmercapto($C_1$-$C_4$), nitro, cyano, alkoxy($C_1$-$C_4$)-carbonyl or alkoxy($C_1$-$C_4$)-carbonyl-alkyl($C_1$-$C_4$), or $R^1$ denotes imidazolyl-alkyl-($C_1$-$C_4$), $R^4$($R^5$)N-$R^6$-, acetylaminoethyl, hydroxy-carbonyl-alkyl$C_1$-$C_4$), 1-(alkoxy($C_1$-$C_4$)-carbonyl)-2-mercapto-ethyl, 1-(alkoxy($C_1$-$C_4$)-carbonyl)-2-hydroxy-ethyl, 1-(alkoxy($C_1$-$C_4$)-carbonyl)-2-alkoxy($C_1$-$C_4$)-ethyl, 1-(alkoxy($C_1$-$C_4$)-carbonyl)-2-alkylthio($C_1$-$C_4$)-ethyl, 1-(alkoxy($C_1$-$C_4$)-carbonyl)-2-(alkyl($C_1$-$C_4$)-carbonyl-thio)-ethyl, 1-(alkoxy($C_1$-$C_4$)-carbonyl)-2-(alkyl($C_1$-$C_4$)-carbonyl-oxy)-ethyl, 1-(alkoxy($C_1$-$C_4$)-carbonyl-2-imidazolyl-ethyl, 1-(alkoxy($C_1$-$C_4$)-carbonyl)-2-indolyl-ethyl or 1-(alkoxy($C_1$-$C_4$)-carbonyl)-2-phenyl-ethyl; $R^2$ and $R^3$ independently of one another denote hydrogen, alkyl($C_1$-$C_5$), alkanoyl($C_1$-$C_5$), alkanoyl($C_2$-$C_5$) which is mono-, di- or trisubstituted by amino, alkoxy($C_1$-$C_4$), hydroxyl, alkanoyloxy($C_1$-$C_4$), phenyl or halogen, benzoyl, benzoyl which is mono- or polysubstituted by halogen, alkoxy($C_1$-$C_4$), alkyl($C_1$-$C_4$), amino, $R^4$($R^5$)N-, hydroxyl, alkanoyloxy($C_1$-$C_4$), mercapto, alkylmercapto($C_1$-$C_4$), nitro, cyano, alkoxy($C_1$-$C_4$)-carbonyl or amido-sulphonyl, pyridylcarbonyl, cycloalkylcarbonyl having 5 to 7 C atoms in the cycloalkyl radical, it being possible for one -$CH_2$-group to be replaced by -O-, -S- or -N($R^{10}$)-; (4-chlorophenoxy)-acetyl; hetero-arylcarbonyl, or $R^2$ and $R^3$ together denote vicinal phenylene-dicarbonyl, phenyl-ene-1-sulphonyl-2-carbonyl, alkylene($C_2$-$C_8$)-dicarbonyl or alkenylene($C_4$-$C_8$)-dicarbonyl, it being possible for the carbon chain of the alkylene- and alkenylene-dicarbonyl also to be interrupted by one or more -O-, -S- or -N($R^4$)-groups, or to contain a spiro-atom, via which a 4-, 5-, 6- or 7-membered ring may be bonded; or (4-chlorophen-oxy)-acetyl; or phenylene-1-sulphinyl-2-carbonyl or phenylene-1-sulphenyl-2-carbonyl; $R^4$ and $R^5$ denote hydrogen or alkyl($C_1$-$C_4$); $R^6$ denotes alkylene($C_1$-$C_4$); $R^7$ and $R^8$ independently of one another denote hydrogen or alkyl($C_1$-$C_4$), or, together with the nitrogen atom to which they are bonded, form a 5- or 6-membered, saturated heterocyclic ring, which may also additionally contain -O- or

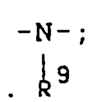

$R^9$ denotes hydrogen, alkyl($C_1$-$C_4$), alkoxy($C_1$-$C_4$), phenyl, alkyl($C_1$-$C_4$)-phenyl or alkoxy-($C_1$-$C_4$)-phenyl; $R^{10}$ denotes hydrogen or alkanoyl($C_1$-$C_5$); and n denotes 1, 2 or 3, or a pharmaceutically acceptable acid addition salt thereof, together with a pharmaceutically acceptable excipient and, if appropriate, pharmaceutically acceptable additives and, if appropriate, also one or more other pharmacological active compounds.

**Claims**

for the Contracting State: AT

1. Process for the preparation of 2-(aminoalkyl)-pyrrole derivatives of the general formula I

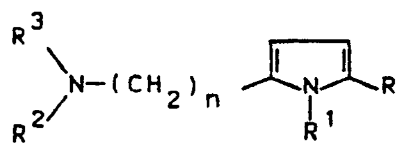 (I)

wherein R denotes hydrogen, alkyl($C_1$-$C_5$) or phenyl; $R^1$ denotes hydrogen, alkyl($C_1$-$C_5$), cyano-alkyl($C_1$-$C_4$) , alkoxy($C_1$-$C_4$)-carbonyl, alkoxy($C_1$-$C_4$)-carbonyl-alkyl-($C_1$-$C_4$), $R^7$($R^8$)N-carbonyl-alkyl($C_1$-$C_4$), phenyl or phenyl-alkyl($C_1$-$C_4$), it being possible for the phenyl or the phenyl of the phenyl-alkyl also to be monosubstituted or polysubstituted by halogen, alkoxy($C_1$-$C_4$), alkyl-($C_1$-$C_4$), $R^4$($R^5$)N-, hydroxyl, mercapto, alkylmercapto-($C_1$-$C_4$), nitro, cyano, alkoxy($C_1$-$C_4$)-carbonyl or alkoxy-($C_1$-$C_4$)-carbonyl-alkyl($C_1$-$C_4$), or $R^1$ denotes imidazolyl-alkyl($C_1$-$C_4$), $R^4$($R^5$)N-$R^6$-, acetyl-amino-ethyl, hydroxy-carbonyl-alkyl($C_1$-$C_4$), 1-(alkoxy($C_1$-$C_4$)carbonyl)-2-mercapto-ethyl, 1-(alkoxy($C_1$-$C_4$)-carbonyl)-2-hydroxy-ethyl, 1-(alkoxy($C_1$-$C_4$)-carbonyl)-2-alkoxy($C_1$-$C_4$)-ethyl, 1-(alkoxy($C_1$-$C_4$)-carbonyl)-2-alkylthio($C_1$-$C_4$)-ethyl, 1-(alkoxy($C_1$-$C_4$)-carbonyl)-2-(alkyl($C_1$-$C_4$)-carbonyl-thio)-ethyl, 1-(alkoxy($C_1$-$C_4$)-carbonyl)-2-(alkyl($C_1$-$C_4$)-carbonyl-oxy)-ethyl, 1-(alkoxy($C_1$-$C_4$)-carbonyl-2-imidazolyl-ethyl, 1-(alkoxy($C_1$-$C_4$)-carbonyl)-2-indolyl-ethyl or 1-(alkoxy($C_1$-$C_4$)-carbonyl)-2-phenyl-ethyl;, $R^2$ and $R^3$ independently of one another denote hydrogen, alkyl($C_1$-$C_5$), alkanoyl($C_1$-$C_5$), alkanoyl($C_2$-$C_5$) which is mono-, di- or trisubstituted by amino, alkoxy($C_1$-$C_4$), hydroxyl, alkanoyl-oxy($C_1$-$C_4$), phenyl or halogen, benzoyl, benzoyl which is mono- or polysubstituted by halogen, alkoxy($C_1$-$C_4$), alkyl($C_1$-$C_4$), amino, $R^4$($R^5$)N-, hydroxy, alkanoyloxy($C_1$-$C_4$), mercapto, alkylmercapto-($C_1$-$C_4$), nitro, cyano, alkoxy($C_1$-$C_4$)-carbonyl or amidosulphonyl, or pyridylcarbonyl, cycloalkylcarbonyl having 5 to 7 C atoms in the cycloalkyl radical, it being possible for one $-CH_2$-group to be replaced by -O-, -S- or -N($R^{10}$)-; (4-chlorophenoxy)-acetyl; or $R^2$ and $R^3$ together denote vicinal phenylenedicarbonyl, phenylene-1-sulphonyl-2-carbonyl, alkylene($C_2$-$C_8$)-dicarbonyl or alkenylene($C_4$-$C_8$)-dicarbonyl, is being possible for the carbon chain of the alkylene- and alkenylene-dicarbonyl also to be interrupted by one or more -O-, -S- or -N($R^4$)-groups, or to contain a spiro-atom, via which a 4-, 5-, 6- or 7-membered ring may be bonded; or phenylene-1-sulphinyl-2-carbonyl or phenylene-1-sulphenyl-2-carbonyl; $R^4$ and $R^5$ denote hydrogen or alkyl($C_1$-$C_4$); $R^6$ denotes alkylene($C_1$-$C_4$); $R^7$ and $R^8$ independently of one another denote hydrogen or alkyl($C_1$-$C_4$), or, together with the nitrogen atom to which they are bonded, form a 5- or 6-membered, saturated heterocyclic ring, which may also additionally contain -O- or

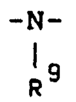 ;

$R^9$ denotes hydrogen, alkyl($C_1$-$C_4$), alkoxy($C_1$-$C_4$), phenyl, alkyl($C_1$-$C_4$)-phenyl or alkoxy($C_1$-$C_4$)-phenyl; $R^{10}$ denotes hydrogen or alkanoyl($C_1$-$C_5$); and n denotes 1, 2 or 3; and their acid addition salts, and in the cases where $R^2$ and $R^3$ together denote 1,2-phenylene-dicarbonyl and at the same time R denotes methyl or ethyl and n denotes the number 1, $R^1$ cannot be ethoxy-carbonyl-methyl, ethoxy-carbonyl or o-methoxy-carbonylphenyl, and, in the cases where $R^2$ and $R^3$ together denote 1,2-phenylene-dicarbonyl and at the same time R denotes methyl or ethyl and n denotes the number 2, $R^1$ cannot be ethoxy-carbonyl or ethoxycarbonylmethyl, and, in the cases where $R^2$ and $R^3$ together denote 1,2-phenylene-dicarbonyl and at the same time n denotes the number 1 and R denotes phenyl, $R^1$ cannot be o-methoxy-carbonylphenyl or ethoxy-carbonylmethyl, characterised in that a 1,4-diketone of the formula II

$(R^2)(R^3)N-(CH_2)_n-CO-CH_2CH_2-CO-R$ (II)

or a 1,4-diketal of the formula IIa

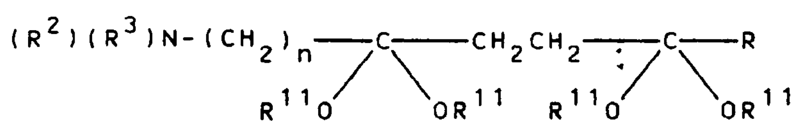

(IIa)

wherein $R^{11}$ denotes alkyl or two adjacent radicals $R^{11}$ together denote alkylene, is reacted with an amine of the formula III

$H_2NR^1$ (III)

in an inert solvent or solvent mixture at temperatures of 0 to 200°C and the compound obtained is converted, if desired, into an acid addition salt in a manner known by itself.

2. Process according to Claim 1, characterised in that the starting compounds are chosen such that compounds of the formula I or acid addition salts thereof are obtained, wherein R denotes methyl.

Process according to Claims 1 and/or 2, characterised in that the starting compounds are chosen such that compounds of the formula I or acid addition salts thereof are obtained, wherein $R^3$ denotes hydrogen, nicotinoyl, 3,4-dimethoxybenzoyl, 4-chlorobenzoyl, 4-chlorophenoxyacetyl or acetyl.

4. Process according to one or several of Claims 1 to 3 characterised in that the starting compounds are chosen such that compounds of the formula I or acid addition salts thereof are obtained, wherein $R^1$ denotes hydrogen, 2-dimethylaminoethyl, 3,4-dimethoxyphenylethyl, 4,5-dimethoxy-2-(methoxycarbonyl)-phenyl, imidazol-4- or -5-yl-ethyl, methoxycarbonylmethyl, acetylamino-ethyl, cyanoethyl, 3-(1-imidazolyl)-propyl or aminocarbonylmethyl.

5. Process according to one or several of Claims 1 to 4, characterised in that the starting compounds are chosen such that the compound 1-(methoxycarbonyl-methyl)-2-(phthalimido-methyl)-5-methyl-pyrrole is obtained.

6. Process according to one or several of Claims 1 to 4, characterised in that the starting-compounds are chosen such that the compound 5-methyl-2-(3,4-dimethoxy-benzoyl-amino-methyl)-pyrrole is obtained.

7. Process according to one or several of Claims 1 to 4, characterised in that the starting compounds are chosen such that the compound 1-(2-methoxy-carbonyl-4,5-dimethoxy-phenyl)-5-methyl-2-(4-chlorophenoxy-acetylamino-methyl)-pyrrole is obtained.

8. Process for preparing pharmaceutical preparations which contain nootropic active substances and which are used for the treatment of humans for combating and preventing-disorders caused by a restriction in cerebral function, or for the treatment of cerebral ageing Processes, characterised in that a 2-(amino-alkyl)-pyrrole derivative of the general formula I

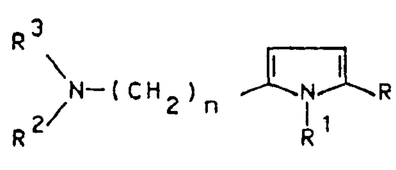

(I)

wherein R denotes hydrogen, alkyl($C_1$-$C_5$) or phenyl; $R^1$ denotes hydrogen, alkyl($C_1$-$C_5$), cyano-alkyl($C_1$-$C_4$), alkoxy($C_1$-$C_4$)-carbonyl, alkoxy($C_1$-$C_4$)-carbonyl-alkyl-($C_1$-$C_4$), $R^7(R^8)N$-carbonyl-alkyl($C_1$-$C_4$), phenyl or phenyl-alkyl($C_1$-$C_4$), it being possible for the phenyl or the phenyl of the phenyl-alkyl also to be monosubstituted or polysubstituted by halogen, alkoxy($C_1$-$C_4$), alkyl-($C_1$-$C_4$), $R^4(R^5)N$-, hydroxyl, mercapto, alkylmercapto-($C_1$-$C_4$), nitro, cyano, alkoxy($C_1$-$C_4$)-carbonyl or alkoxy-($C_1$-$C_4$)-carbonyl-alkyl($C_1$-$C_4$), or $R^1$ denotes imidazolyl-alkyl($C_1$-$C_4$), $R^4(R^5)N$-$R^6$-, acetyl-amino-ethyl, hydroxy-carbonyl-alkyl($C_1$-$C_4$), 1-(alkoxy($C_1$-$C_4$)-carbonyl-2-mercapto-ethyl, 1-(alkoxy($C_1$-$C_4$)-carbonyl)-2-hydroxy-ethyl, 1-(alkoxy($C_1$-$C_4$)-carbonyl)-2-alkoxy($C_1$-$C_4$)-ethyl, 1-(alkoxy($C_1$-$C_4$)-carbonyl)-2-alkylthio($C_1$-$C_4$)-ethyl, 1-(alkoxy($C_1$-$C_4$)-carbonyl)-2-(alkyl($C_1$-$C_4$)-carbonyl-thio)-ethyl, 1-(alkoxy($C_1$-$C_4$)-carbonyl)-2-(alkyl($C_1$-$C_4$)-carbonyl-oxy)-ethyl, 1-(alkoxy($C_1$-$C_4$)-carbonyl)-2- imidazolyl-ethyl, 1-(alkoxy($C_1$-$C_4$)-carbonyl-2-indolyl-ethyl or 1-(alkoxy($C_1$-$C_4$)-carbonyl)-2-phenyl-ethyl; $R^2$ and $R^3$

independently of one another denote hydrogen, alkyl($C_1$-$C_5$) alkanoyl($C_1$-$C_5$), alkanoyl($C_2$-$C_5$), which is mono-, di- or trisubstituted by amino, alkoxy($C_1$-$C_4$), hydroxyl, alkanoyloxy($C_1$-$C_4$), phenyl or halogen, benzoyl, benzoyl which is mono- or polysubstituted by halogen, alkoxy($C_1$-$C_4$), alkyl($C_1$-$C_4$), amino, $R^4(R^5)N$-, hydroxyl, alkanoyloxy($C_1$-$C_4$), mercapto, alkylmercapto($C_1$-$C_4$), nitro, cyano, alkoxy($C_1$-$C_4$)-carbonyl- or amidosulphonyl, or pyridylcarbonyl, cycloalkylcarbonyl having 5 to 7 C atoms in the cycloalkyl radical, it being possible for one -$CH_2$-group to be replaced by -O-, -S-, or -N($R^{10}$)-; (4-Chlorophenoxy)-acetyl; or $R^2$ and $R^3$ together denote vicinal phenylene-dicarbonyl, phenylene-1-sulphonyl-2-carbonyl, alkylene($C_2$-$C_8$)-dicarbonyl or alkenylene-($C_4$-$C_8$)-dicarbonyl, it being possible for the carbon chain of the alkylene- and alkenylene-dicarbonyl also to be interrupted by one or more -O-, -S- or -N($R^4$)-groups, or to contain a spiro atom, via which a 4-, 5-, 6- or 7-membered ring may be bonded; or phenylene-1-sulphinyl-2-carbonyl or phenylene-1-sulphenyl-2-carbonyl; $R^4$ and $R^5$ denote hydrogen or alkyl($C_1$-$C_4$); $R^6$ denotes alkylene-($C_1$-$C_4$); $R^7$ and $R^8$ independently of one another denote hydrogen or alkyl($C_1$-$C_4$), or, together with the nitrogen atom to which they are bonded, form a 5- or 6-membered, saturated heterocyclic ring, which may also additionally contain -O- or -N-; $R^9$ denotes hydrogen, alkyl($C_1$-$C_4$), alkoxy($C_1$-$C_4$), phenyl, alkyl($C_1$-$C_4$)-phenyl or alkoxy-($C_1$-$C_4$)-phenyl; $R^{10}$ denotes hydrogen or alkanoyl($C_1$-C5)) and n denotes 1,2 or 3, or a pharmacQlogically acceptable acid addition salt thereof is prepared as an active substance in that a 1,4-diketone of the formula II

$(R^2)(R^3)N\text{-}(CH_2)\text{ -}CO\text{-}CH_2CH_2\text{-}CO\text{-}R$ (II)

or a 1,4-diketal of the formula IIa

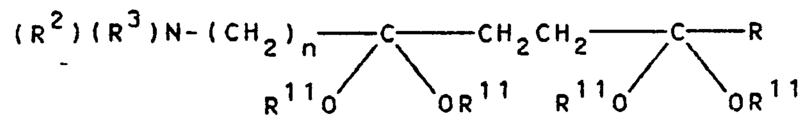

(IIa)

wherein $R^{11}$ denotes alkyl or two adjacent radicals $R^{11}$ together denote alkylene, is reacted with an amine of the formula III

$H_2NR^1$ (III)

in an inert solvent or solvent mixture at temperatures of 0 to 200°C and the compound is converted, if desired, into an acid addition salt and that the compound of formula I or its acid addition salt is isolated and that the active substance thus obtained is converted in a manner known by itself by mixing with one or several pharmaceutically acceptable carriers into an administrable pharmaceutical product.

**Revendications**

pour les Etats contractants: DE, BE, FR, GB, IT, NL, SE, CH.

1. (Aminoalkyl)-2 pyrroles, de formule générale I :

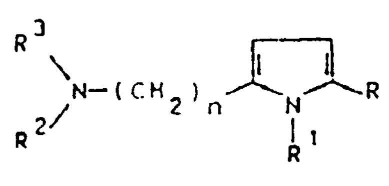

(I)

dans laquelle

R représente un atome d'hydrogène, un groupe alkyle (en $C_1$ à $C_5$), un groupe phényle;

$R^1$ un atome d'hydrogène, un groupe alkyle (en $C_1$ à $C_5$) groupe cyano-alkyle (en $C_1$ à $C_4$) $_{alcoxy}$ (en $C_1$ à $C_4$)-carbonyle, alcoxy (en $C_1$ à $C_4$)-carbonyl-alkyle (en $C_1$ à $C_4$), $R^7(R^8)N$-carbonyl-alkyle (en $C_1$ à $C_4$), phenyle, phenyl-alkyle (en $C_1$ à $C_4$), (le groupe phényle ou le reste phényle du groupe phényl-alkyle pouvant aussi être

substitué, une ou plusieurs fois, également par de l'halogène, par un groupe alcoxy (en $C_1$ à $C_4$), alkyle (en $C_1$ à $C_4$), $R^4(R^5)N$-, hydroxy, mercapto, alkylmercapto (en $C_1$ à $C_4$), nitro, cyano, alcoxy (en $C_1$ à $C_4$)-carbonyle alcoxy (en $C_1$ à $C_4$)-carbonyl-alkyle (en $C_1$ à $C_4$), imidazolyl-alkyle (en $C_1$ à $C_4$), $R^4(R^5)N-R^6$, acétyl-amino-éthyle, hydroxy-carbonyl-alkyle (en $C_1$ à $C_4$), (alcoxy (en $C_1$ à $C_4$)-carbonyl)-1 mercapto-2 éthyle, (alcoxy (en $C_1$ à $C_4$)-carbonyl)-1 hydroxy-2 éthyle, (alcoxy (en $C_1$ à $C_4$)-carbonyl)-1-alcoxy-2 (en $C_1$ à $C_4$)-éthyle, (alcoxy (en $C_1$ à $C_4$)-carbonyl)-1 alkylthio (en $C_1$ à $C_4$)-2 éthyle, (alcoxy (en $C_1$ à $C_4$)-carbonyl)-1 (alkyl (en $C_1$ à $C_4$)-carbonyl)-thio)-2 éthyle, (alcoxy (en $C_1$ à $C_4$)-carbonyl)-1 (alkyl (en $C_1$ à $C_4$)-carbonyl-oxy)-2 éthyle, alcoxy (en $C_1$ à $C_4$)-carbonyl)-1 imidazolyl-2 éthyle, (alcoxy (en $C_1$ à $C_4$)-carbonyl)-1 indolyl)-2 éthyle, (alcoxy (en $C_1$ à $C_4$)-carbonyl)-1 phényl-2 éthyle ;

$R^2$ et $R^3$ représentent chacun, indépendamment l'un de l'autre, un atome d'hydrogène, un groupe alkyle (en $C_1$ à $C_5$), alcanoyle (en $C_1$ à $C_5$), alcanoyle (en $C_2$ à $C_5$) ; (substitué une, deux ou trois fois par un groupe amino, alcoxy (en $C_1$ à $C_4$), hydroxy, alcanoyloxy (en $C_1$ à $C_4$), phényle ou halogéno), un groupe benzoyle, un groupe benzoyle (substitué une ou plusieurs fois par un atome d'halogène, un groupe alcoxy (en $C_1$ à $C_4$), alkyle (en $C_1$ à $C_4$), amino, $R^4(R^5)N$-, hydroxy, alcanoyloxy (en $C_1$ à $C_4$) mercapto alkylmercapto (en $C_1$ à $C_4$), nitro, cyano, alcoxy (en $C_1$ à $C_4$)- carbonyle, amidosulfonyle), un groupe pyridyl-carbonyle, cycloalkyl-carbonyle ayant 5 à 7 atomes de carbone dans le reste cycloalkyle, un groupe $-CH_2$ pouvant être également remplacé par -O-, -S- ou $-N(R^{10})$- ; un groupe (chloro-4 phénoxy)-acétyle ; ou bien $R^2$ et $R^3$ forment ensemble un groupe phénylène dicarbonyle vicinal, phénylène-sulfonyl-1 carbonyle-2,alkylène (en $C_2$ a $C_8$)-dicarbonyle, alcénylène (en $C_4$ à $C_6$)-dicarbonyle, la chaine carbonée du groupe alkylène- ou alcénylène-dicarbonyle pouvant également être interrompue une ou plusieurs fois par -O-, ou -S-, $-N(R^4)$- ou pouvant contenir un atome de type spiro, par l'intermédiaire duquel peut être fixé un noyau en position 4, 5, 6 ou 7 ; un groupe phénylène-sulfinyl-1 carbonyle-2 phénylène-sulfonyl-1 carbonyl

$R^4$ et $R^5$ représentent chacun un atome d'hydrogène ou un groupe alkyle (en $C_1$ à $C_4$);

$R^6$ représente un groupe alkylène (en $C_1$ à $C_4$)

$R^7$ et $R^8$ représentent, indépendamment l'un de l'autre, un atome d'hydrogène, un groupe alkyle (en $C_1$ à $C_4$) ou avec l'atome d'azote auquel ils sont reliés, ils forment un noyau hétérocyclique saturé pentagonal ou hexagonal, qui peut aussi contenir -O- ou

-N- ;
|
R9

$R^9$ représente un atome d'hydrogène, un groupe alkyle (en $C_1$ à $C_4$), alcoxy (en $C_1$ à $C_4$), phényle, alkyle (en $C_1$ à $C_4$)-phényle, alcoxy (en $C_1$ à $C_4$)-phényle ;

$R^{10}$ représente un atome d'hydrogène, un groupe alcanoyle (en $C_1$ à $C_5$);

n vaut 1, 2 ou 3 ;

ainsi que leurs sels d'addition d'acide, et, si $R^2$ et $R^3$ pris ensemble représentent un groupe phénylène-dicarbonyle-1,2 et qu'en même temps R représente un groupe méthyle ou éthyle et n vaut 1, $R^1$ ne peut pas représenter un groupe éthoxy-carbonylméthyle, éthoxy-carbonyle ou o-methoxy-carbonylphenyle, et, si $R^2$ et $R^3$ représentent ensemble un groupe phénylène-dicarbonyl-1, 2 et simultanément R est un groupe méthyle ou éthyle et n vaut 2, $R^1$ ne peut pas représenter un groupe éthoxy-carbonyle ou ethoxy-carbonylmethyle, et, si $R^2$ et $R^3$ représentent ensemble un groupe phénylène-dicarbonyle 1,2 et simultanément n vaut 1 et R représente un groupe phényle, $R^1$ ne peut pas représenter un groupe -o-méthoxy-carbonylphényle ou éthoxy-carbonylméthyle.

2. Composés selon la revendication 1 caractérisés en ce que R représente un groupe méthyle.

3. Composés selon la revendication 1 et/ou 2 caractérisés en ce que $R^3$ représente un atome d'hydrogène, un groupe nicotinoyle, diméthoxy-3,4 benzoyle, chloro-4 benzoyle, chloro-4 phénoxyacétyle, acétyle.

4. Composés selon l'une ou plusieurs des revendications 1 à 3 caractérisés en ce que $R^1$ représente un atome d'hydrogène, un groupe diméthylamino-2 éthyle, diméthoxy-3,4 phényléthyle, diméthoxy-3,5 (méthoxycarbonyl)-2 phényle, (imidazolyl-4 -5))-éthyle, méthoxy carbonyl-méthyle, acétylamino-éthyle, cyanoéthyle, (imidazolyl-1)-3 propyle, aminocarbonylméthyle.

5. Le (méthoxy-carbonylméthyl)-1 (phthalimido-méthyl)-2 méthyl-5 pyrrole.

6. Le méthyl-5 (diméthoxy-3,4 benzoylamino-méthyl)-2 pyrrole.

7. Le (méthoxy-carbonyl)-2 diméthoxy-4,5 phényl)-1 méthyl-5 (chloro-4 phénoxy-acgtylaminométhyl)-2 pyrrole.

6. Procédé pour préparer les composés de formule I selon une ou plusieurs des revendications 1 à 7, caractérisé en ce qu'on fait réagir une dicétone-1,4 de formule II

$$(R^2)(R^3)N-(CH_2)_n-CO-CH_2CH_2-CO-R \qquad (II)$$

ou un dicétal-1,4 de formule IIa

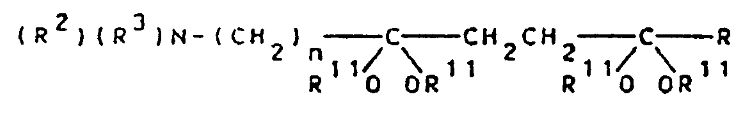

(IIa)

(dans laquelle $R^{11}$ représente un groupe alkyle ou deux radicaux $R^{11}$ voisins forment ensemble un groupe alkylène) avec une amine de formule III

$H_2NR^1$

(dans laquelle R, $R^1$, $R^2$ et $R^3$ ont les sens indiqués aux revendications 1 à 4) dans un solvant ou un mélange de solvants inerte à des températures de 0 à 200°C et l'on transforme éventuellement le composé résultant, de façon connue en soi, en un sel d'addition d'acide .

9. Utilisation d'un (aminoalkyl)-2 pyrrole de formule générale I :

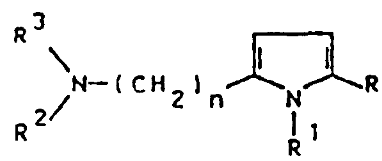

(I)

dans laquelle

R représente un atome d'hydrogène, un groupe alkyle (en $C_1$ à $C_5$) un phényle ;

$R_1$ un atome d'hydrogène, un groupe alkyle (en $C_1$ à $C_5$), groupe cyano-alkyle (en $C_1$ à $C_4$), alcoxy (en $C_1$ à $C_4$)-carbonyle, alcoxy (en $C_1$ à $C_4$)-carbonyl-alkyle (en $C_1$ à $C_4$) $R^7(R^8)$N-carbonyl-alkyle (en $C_1$ à $C_4$), phényle, phényl-alkyle (en $C_1$ à $C_4$), (le groupe phényle ou le reste phényle du groupe phényl-alkyle pouvant aussi être substitué, une ou plusieurs fois, également par de l'halogène, par un groupe alcoxy (en $C_1$ à $C_4$), alkyle (en $C_1$ à $C_4$), $R^4(R^5)$N-, hydroxy, mercapto, alkylmercapto (en $C_1$ à $C_4$), nitro, cyano, alcoxy (en $C_1$ à $C_4$)-carbonyle, alcoxy (en $C_1$ à $C_4$)-carbonyl-alkyle (en $C_1$ à $C_4$), imidazolyl-alkyle (en $C_1$ à $C_4$) $R^4(R^5)N-R^6$ acétyl-amino-éthyle, hydroxy-carbonyl-alkyle (en $C_1$ à $C_4$), (alcoxy (en $C_1$ à $C_4$)-carbonyl)-1 mercapto-2 éthyle, (alcoxy (en $C_1$ à $C_4$)-carbonyl)-1 hydroxy-2 éthyle, (alcoxy (en $C_1$ à $C_4$)-carbonyl)-1-alcoxy-2 (en $C_1$ à $C_4$)-éthyle, (alcoxy (en $C_1$ à $C_4$)-carbonyl)-1 alkylthio (en $C_1$ à $C_4$)-2 éthyle, (alcoxy (en $C_1$ à $C_4$)-carbonyl)-1 (alkyl (en $C_1$ à $C_4$)-carbonyl-thio)-2 éthyle, (alcoxy (en $C_1$ à $C_4$)-carbonyl)-1 (alkyl (en $C_1$ à $C_4$)-carbonyl-oxy)-2 éthyle, alcoxy (en $C_1$ à $C_4$)-carbonyl)-1 imidazolyl-2 éthyle, (alcoxy (en $C_1$ à $C_4$)-carbonyl)-1 indolyl)-2 éthyle, (alcoxy (en $C_1$l à $C_4$)-carbonyl)-1 phényl-2 éthyle ;

$R^2$ et $R^3$ représentent chacun, indépendamment l'un de l'autre, un atome d'hydrogène, un groupe alkyle (en $C_1$ à $C_5$) alcanoyle (en $C_1$ à $C_5$) alcanoyle (en $C_2$ à $C_5$) . (substitué une, deux ou trois fois par un groupe amino, alcoxy (en $C_1$ à $C_4$), hydroxy, alcanoyloxy (en $C_1$ à $C_4$), phényle ou halogéno), un groupe benzoyle, un groupe benzoyle (substitué une ou plusieurs fois par un atome d'halogène, un groupe alcoxy (en $C_1$ à $C_4$) alkyle (en $C_1$ à $C_4$), amino, $R^4(R^5)$N-, hydroxy, alcanoyloxy (en $C_1$ à $C_4$) mercapto, alkylmercapto (en $C_1$ à $C_4$) nitro cyano

alcoxy (en $C_1$ à $C_4$)- carbonyle amidosulfonyle) un groupe pyridyl-carbonyle, cycloalkyl-carbonyle ayant 5 à 7 atomes de carbone dans le reste cycloalkyle, un groupe -$CH_2$ pouvant être également remplacé par -O-, -S- ou -N($R^{10}$)- ; un groupe (chloro-4 phénoxy)-acétyle ; ou bien $R^2$ et $R^3$ forment ensemble un groupe phénylène dicarbonyle vicinal, phénylène-sulfonyl-1 carbonyle-2 alkylène (en $C_2$ a $C_8$)-dicarbonyle, alcénylène (en $C_4$ à $C_8$)-dicarbonyle, la chaîne carbonée du groupe alkylène- ou alcénylène-dicarbonyle pouvant également être interrompue une ou plusieurs fois par -O-, ou -S-, -N($R^4$)- ou pouvant contenir un atome de type spiro, par l'intermédiaire duquel peut être fixé un noyau en position 4, 5, 6 ou 7 ; un groupe phénylène-sulfinyl-1 carbonyl-2, phénylène-sulfényl-1 carbonyle-2 ;

$R^4$ et $R^5$ représentent chacun un atome d'hydrogène ou un groupe alkyle (en $C_1$ à $C_4$);

$R^6$ représente un groupe alkylène (en $C_1$ à $C_4$);

$R^7$ et $R^8$ représentent, indépendamment l'un de l'autre, un atome d'hydrogène, un groupe alkyle (en $C_1$ à $C_4$), ou, avec l'atome d'azote auquel ils sont reliés, ils forment un noyau hétérocyclique saturé pentagonal ou hexagonal, qui peut aussi contenir -O- ou

-N- ;
|
$R^9$

$R^9$ représente un atome d'hydrogène, un groupe alkyle (en $C_1$ à $C_4$), alcoxy (en $C_1$ à $C_4$), phényle, alkyle (en $C_1$ à $C_4$)-phényle, alcoxy (en $C_1$ à $C_4$)-phényle;

$R^{10}$ représente un atome d'hydrogène, un groupe alcanoyle (en $C_1$ à $C_5$);

n vaut 1, 2 ou 3 ;

ou d'un sel d'addition d'acide, pharmacologiquement acceptable, pour préparer un médicament pour combattre et éviter des maladies provoquées par une limitation du fonctionnement cérébral, ou pour traiter des processus d'altération cérébrale.

10. Préparation pharmaceutique, caractérisée en ce qu'elle contient comme substance active un (aminoalkyl)-2 pyrrole de formule générale 1 :

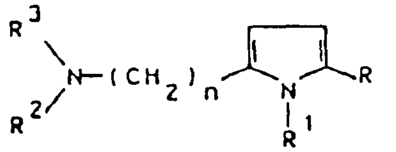

(I)

dans laquelle

R représente un atome d'hydrogène, un groupe alkyle (en $C_1$ à $C_5$) un phényle;

$R^1$ un atome d'hydrogène, un groupe alkyle (en $C_1$ à $C_5$), groupe cyano-alkyle (en $C_1$ à $C_4$), alcoxy (en $C_1$ à $C_4$)-carbonyle, alcoxy (en $C_1$ à $C_4$)-carbonyl-alkyle (en $C_1$ à $C_4$) $R^7$($R^8$)N-carbonyl-alkyle (en $C_1$ à $C_4$), phényle, phényl-alkyle (en $C_1$ à $C_4$), (le groupe phényle ou le reste phényle du groupe phényl-alkyle pouvant aussi être substitué, une ou plusieurs fois, également par de l'halogène, par un groupe alcoxy (en $C_1$ à $C_4$), alkyle (en $C_1$ à $C_4$), $R^4$($R^5$)N-, hydroxy, mercapto, alkylmercapto (en $C_1$ à $C_4$), nitro, cyano, alcoxy (en $C_1$ à $C_4$)-carbonyle alcoxy (en $C_1$ à $C_4$)-carbonyl-alkyle (en $C_1$ à $C_4$), imidazolyl-alkyle (en $C_1$ à $C_4$), $R^4$($R^5$)N-$R^6$, acétyl-amino-éthyle, hydroxy-carbonyl-alkyle (en $C_1$ à $C_4$), (alcoxy (en $C_1$ à $C_4$)-carbonyl)-1 mercapto-2 éthyle, (alcoxy (en $C_1$ à $C_4$)-carbonyl)-1 hydroxy-2 éthyle, (alcoxy (en $C_1$ à $C_4$)-carbonyl)-1-alcoxy-2 (en $C_1$ à $C_4$)-éthyle, (alcoxy (en $C_1$ à $C_4$)-carbonyl)-1 alkylthio (en $C_1$ à $C_4$)-2 éthyle, (alcoxy (en $C_1$ à $C_4$)-carbonyl)-1 (alkyl (en $C_1$ à $C_4$)-carbonyl-thio)-2 éthyle (alcoxy (en $C_1$ à $C_4$)-carbonyl)-1 (alkyl (en $C_1$ à $C_4$)-carbonyl-oxy)-2 éthyle, alcoxy (en $C_1$ à $C_4$)-carbonyl)-1 imidazolyl-2 éthyle, (alcoxy (en $C_1$ à $C_4$)-carbonyl)-1 indolyl)-2 éthyle, (alcoxy (en $C_1$ à $C_4$)-carbonyl)-1 phényl-2 éthyle;

$R^2$ et $R^3$ représentent chacun, indépendamment l'un de l'autre, un atome d'hydrogène un groupe alkyle (en $C_1$ à $C_5$), alcanoyle (en $C_1$ à $C_5$), alcanoyle (en $C_2$ à $C_5$); (substitué une, deux ou trois fois par un groupe amino, alcoxy (en $C_1$ à $C_4$), hydroxy, alcanoyloxy (en $C_1$ à $C_4$), phényle ou halogéno), un groupe benzoyle, un groupe benzoyle (substitué une ou plusieurs fois par un atome d'halogène, un groupe alcoxy (en $C_1$ à $C_4$) alkyle (en $C_1$ à $C_4$) amino $R^4$($R^5$)N-, hydroxy, alcanoyloxy (en $C_1$ à $C_4$), mercapto, alkylmercapto (en $C_1$ à $C_4$), nitro, cyano, alcoxy (en $C_1$ à $C_4$)- carbonyle, amidosulfonyle), un groupe pyridyl-carbonyle, cycloalkyl-carbonyle ayant 5 à 7 atomes de carbone dans le reste cycloalkyle, un groupe -$CH_2$ pouvant être également remplacé par -O-, -S- ou -N($R^{10}$)- ; un groupe (chloro-4 phénoxy)-acétyle ; ou bien $R^2$ et $R^3$ forment ensemble un groupe phénylène dicarbonyle vicinal, phénylène-sulfényl-1 carbonyle-2 alkylène (en $C_2$ a $C_8$)-dicarbonyle, alcénylène (en $C_4$ à $C_8$)-dicarbonyle, la chaîne carbonée du groupe alkylène- ou alcénylène-dicarbonyle pouvant également être interrompue une ou plusieurs fois par -O-, ou -S-, -N($R^4$)- ou pouvant contenir un atome de type spiro, par l'intermédiaire duquel peut être fixé un noyau en position 4, 5, 6 ou 7 ; un groupe phénylène-sulfényl-1 carbonyle-2, phénylène-sulfonyl-1 carbonyle-2;

$R^4$ et $R^5$ représentent chacun un atome d'hydrogène ou un groupe alkyle (en $C_1$ à $C_4$) .

$R^6$ représente un groupe alkylène (en $C_1$ à $C_4$) ;

$R^7$ et $R^8$ représentent, indépendamment l'un de l'autre, un atome d'hydrogène, un groupe alkyle (en $C_1$ à $C_4$), ou, avec l'atome d'azote auquel ils sont reliés, ils forment un noyau hétérocyclique saturé pentagonal ou hexagonal, qui peut aussi contenir -O- ou

-N- ;
I
R9

$R^9$ représente un atome d'hydrogène, un groupe alkyle (en $C_1$ à $C_4$), alcoxy (en $C_1$ à $C_4$), phényle, alkyle (en $C_1$ à $C_4$)-phényle, alcoxy (en $C_1$ à $C_4$)-phényle ;

$R^{10}$ représente un atome d'hydrogène, un groupe alcanoyle (en $C_1$ à $C_5$);

n vaut 1, 2 ou 3;

ou un de ses sels d'addition d'acides pharmaceutiquement acceptables, avec un excipient ou véhicule pharmaceutiquement acceptable et éventuellement des additifs ou excipients pharmaceutiquement acceptables et éventuellement encore une ou plusieurs autres substances pharmacologiques.

**Revendications**

pour l'Etat contractant: AT.

1. Procédé pour préparer des (aminoalkyl)-2 pyrroles,de formule générale I:

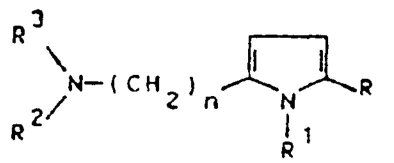

(I)

dans laquelle

R représente un atome d'hydrogène, un groupe alkyle (en $C_1$ à $C_5$) un groupe phényle ,

$R^1$ un atome d'hydrogène, un groupe alkyle (en $C_1$ à $C_5$), groupe cyano-alkyle (en $C_1$ à $C_4$), alcoxy (en $C_1$ à $C_4$)-carbonyle, alcoxy (en $C_1$ à $C_4$)-carbonyl-alkyle (en $C_1$ à $C_4$), $R^7(R^8)$N-carbonyl-alkyle (en $C_1$ à $C_4$), phényle, phényl-alkyle (en $C_1$ à $C_4$ (le groupe phényle ou le reste phényle du groupe phényl-alkyle pouvant aussi être substitué, une ou plusieurs fois, également par de l'halogène, par un groupe alcoxy (en $C_1$ à $C_4$), alkyle (en $C_1$ à $C_4$), $R^4(R^5)$N-, hydroxy, mercapto, alkylmercapto (en $C_1$ à $C_4$) nitro, cyano, alcoxy (en $C_1$ à $C_4$)-carbonyle alcoxy (en $C_1$ à $C_4$)-carbonyl-alkyle (en $C_1$ à $C_4$), imidazolyl-alkyle (en $C_1$ à $C_4$), $R^4(R^5)$N-$R^6$, acétyl-amino-éthyle, hydroxy-carbonyl-alkyle (en $C_1$ à $C_4$) (alcoxy (en $C_1$ à $C_4$)-carbonyl)-1 mercapto-2 éthyle, (alcoxy (en $C_1$ à $C_4$)-carbonyl)-1 hydroxy-2 éthyle, (alcoxy (en $C_1$ à $C_4$)-carbonyl)-1-alcoxy-2 (en $C_1$ à $C_4$)-éthyle, (alcoxy (en $C_1$ à $C_4$)-carbonyl)-1 alkylthio (en $C_1$ à $C_4$)-2 éthyle, (alcoxy (en $C_1$ à $C_4$)-carbonyl)-1 (alkyl (en $C_1$ à $C_4$)-carbonyl-thio)-2 éthyle (alcoxy (en $C_1$ à $C_4$)-carbonyl)-1 (alkyl (en $C_1$ à $C_4$)-carbonyl-oxy)-2 éthyle, alcoxy (en $C_1$ à $C_4$)-carbonyl)-1 imidazolyl-2 éthyle, (alcoxy (en $C_1$ à $C_4$)-carbonyl)-1 indolyl)-2 éthyle, (alcoxy (en $C_1$ à $C_4$)-carbonyl)-1 phényl-2 éthyle ;

$R^2$ et $R^3$ représentent chacun indépendamment l'un de l'autre, un atome d'hydrogène, un groupe alkyle (en $C_1$ à $C_5$) alcanoyle (en $C_1$ à $C_5$) alcanoyle (en $C_2$ à $C_5$); (substitués une, deux ou trois fois par un groupe amino, alcoxy (en $C_1$ à $C_4$) hydroxy alcanoyloxy (en $C_1$ à $C_4$), phényle ou halogéno), un groupe benzoyle, un groupe benzoyle (substitué une ou plusieurs fois par un atome d'halogène, un groupe alcoxy (en $C_1$ à $C_4$) alkyle (en $C_1$ à $C_4$) amino $R^4(R^5)$N-, hydroxy, alcanoyloxy (en $C_1$ à $C_4$) mercapto, alkylmercapto (en $C_1$ à $C_4$), nitro, cyano, alcoxy (en $C_1$ à $C_4$)- carbonyle, amidosulfonyle), un groupe pyridyl-carbonyle, cycloalkyl-carbonyle ayant 5 à 7 atomes de carbone dans le reste cycloalkyle, un groupe $-CH_2$ pouvant être également remplacé par -O-, -S- ou -N($R^{10}$)- ; un groupe (chloro-4 phénoxy)-acétyle ; ou bien $R^2$ et $R^3$ forment ensemble un groupe phénylène dicarbonyle vicinal, phénylène-sulfényl-1 carbonyle-2,alkylène (en $C_2$ à $C_8$)-dicarbonyle, alcénylène (en $C_4$ à $C_8$)-dicarbonyle, la chaîne carbonée du groupe alkylène- ou alcénylène-dicarbonyle pouvant également être interrompue une ou plusieurs fois par -O-, ou -S-, -N($R^4$)- ou pouvant contenir un atome de type spiro, par l'intermédiaire duquel peut être fixé un noyau en position 4, 5, 6 ou 7 ; un groupe phénylène-sulfinyl-1 carbonyl-2, phénylène-sulfényl-1 carbonyle-2 ;

$R^4$ et $R^5$ représentent chacun un atome d'hydrogène ou un groupe alkyle (en $C_1$ à $C_4$);

$R^6$ représente un groupe alkylène (en $C_1$ à $C_4$);

$R^7$ et $R^8$ représentent, indépendamment l'un de l'autre, un atome d'hydrogène, un groupe alkyle (en $C_1$ à $C_4$) ou avec l'atome d'azote auquel ils sont reliés, ils forment un noyau hétérocyclique saturé pentagonal ou hexagonal, qui peut aussi contenir -O- ou

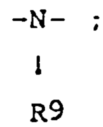 ;

$R^9$ représente un atome d'hydrogène, un groupe alkyle (en $C_1$ à $C_4$) alcoxy (en $C_1$ à $C_4$), phényle, alkyle (en $C_1$ à $C_4$)-phényle, alcoxy (en $C_1$ à $C_4$)-phényle ;

$R^{10}$ représente un atome d'hydrogène, un groupe alcanoyle (en $C_1$ à $C_5$);

n vaut 1, 2 ou 3),

caractérisés en ce qu'on fait réagir la dicétone de formule II

$$(R^2)(R^3)N-(CH_2)_n-CO-CH_2CH_2-CO-R \qquad (II)$$

ou un dicétal-1,4 de formule IIa

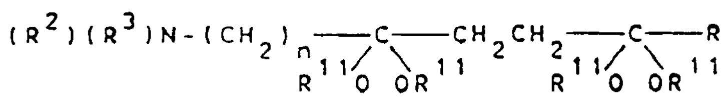 (IIa)

(dans laquelle $R^{11}$ représente un groupe alkyle ou deux radicaux $R^{11}$ voisins forment ensemble un groupe alkylène) avec une amine de formule III

$H_2NR^1$

dans un solvant ou un mélange de solvants inerte, à des températures de 0 à 200°C et l'on transforme éventuellement le composé résultant, de façon connue en soi, en un sel d'addition d'acide.

2. Procédé selon la revendication 1, caractérisé en ce qu'on choisit les composés de départ de manière qu'il en résulte des composés de formule I ou leurs sels d'addition d'acides, dans lesquels R représente un groupe méthyle.

3. Procédé selon la revendication 1 et/ou 2, caractérisé en ce qu'on choisit les composés de départ de manière qu'il en résulte des composés de formule I ou leurs sels d'addition d'acides, dans lesquels $R^3$ représente un atome d'hydrogène, un groupe nicotinoyle, diméthoxy-3,4 benzoyle, chloro-4 benzoyle, chloro-4 phénoxyacétyle ou acétyle.

4. Procédés selon l'une ou plusieurs des revendications 1 à 3 caractérisés en ce qu'on choisit les composés de départ de manière qu'il en résulte des composés de formule I ou leurs sels d'addition d'acides, dans lesquels $R^1$ représente un atome d'hydrogène, un groupe diméthylamino-2 éthyle, diméthoxy-3,4 phényléthyle, diméthoxy-4,5 (méthoxy-carbonyl)-2 phényle, (imidazolyl-4)-5))-éthyle, méthoxy-carbonylméthyle, acétylamino-éthyle, cyanéthyle, (imidazolyl-1)-3 propyle, aminocarbonylméthyle.

5. Procédé selon l'une quelconque des revendications 1 à 4, caractérisé en ce qu'on choisit les composés de départ de manière qu'il en résulte le composé (méthoxy-carbonylméthyl)-1 (phtalimido méthyl)-2 méthyl-5 pyrrole.

6. Procédé selon l'une ou plusieurs des revendications 1 à 4, caractérisé en ce qu'on choisit les composés de

départ de manière qu'il en résulte le composé méthyl-5 (diméthoxy-3,4 benzoylaminométhyl)-2 pyrrole.

7. Procédé selon l'une ou plusieurs des revendications 1 à 4, caractérisé en ce qu'on choisit les composés de départ de manière qu'il en résulte le composé (méthoxy-carbonyl-2 diméthoxy-4,5 phényl)-1 méthyl-5 (chloro-4 phénoxyacétylaminométhyl)-2 pyrrole.

8. Procédé pour fabriquer des préparations pharmaceutiques, qui contiennent des substances actives psychotropes et que l'on utilise pour traiter les êtres humains pour combattre et empêcher des maladies provoquées par une limitation du fonctionnement cérébral ou pour traiter les processus d'altération cérébrale, procédé caractérisé en ce qu'on prépare un (aminnalkyl)-2 pyrrole de formule générale I :

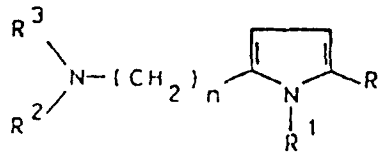

(I)

(dans laquelle

R représente un atome d'hydrogène, un groupe alkyle (en $C_1$ à $C_5$), un phényle;

$R^1$ un atome d'hydrogène, un groupe alkyle (en $C_1$ à $C_5$), groupe cyano-alkyle (en $C_1$ à $C_4$), alcoxy (en $C_1$ à $C_4$)-carbonyle, alcoxy (en $C_1$ à $C_4$)-carbonyl-alkyle (en $C_1$ à $C_4$) $R^7(R^8)$N-carbonyl-alkyle (en $C_1$ à $C_4$) phényle, phényl-alkyle (en $C_1$ à $C_4$) (le groupe phényle ou le reste phényle du groupe phényl-alkyle pouvant aussi être substitué, une ou plusieurs fois, également par de l'halogène, par un groupe alcoxy (en $C_1$ à $C_4$), alkyle (en $C_1$ à $C_4$), $R^4(R^5)$N-, hydroxy, mercapto, alkylmercapto (en $C_1$ à $C_4$) nitro, cyano alcoxy (en $C_1$ à $C_4$)-carbonyle, alcoxy (en $C_1$ à $C_4$)-carbonyl-alkyle (en $C_1$ à $C_4$), imidazolyl-alkyle (en $C_1$ à $C_4$), $R^4(R^5)N-R^6$, acétyl-amino-éthyle, hydroxy-carbonyl-alkyle (en $C_1$ à $C_4$) (alcoxy (en $C_1$ à $C_4$)-carbonyl)-1 mercapto-2 éthyle, (alcoxy (en $C_1$ à $C_4$)-carbonyl)-1 hydroxy-2 éthyle, (alcoxy (en $C_1$ à $C_4$)-carbonyl)-1-alcoxy-2 (en $C_1$ à $C_4$)-éthyle, (alcoxy (en $C_1$ à $C_4$)-carbonyl)-1 alkylthio (en $C_1$ à $C_4$)-2 éthyle;, (alcoxy (en $C_1$ à $C_4$)-carbonyl)-1 (alkyl (en $C_1$ à $C_4$)-carbonyl-thio)-2 éthyle, (alcoxy (en $C_1$ à $C_4$)-carbonyl)-1 (alkyl (en $C_1$ à $C_4$)-carbonyl-oxy)-2 éthyle, (alcoxy (en $C_1$ à $C_4$)-carbonyl)-1 imidazolyl-2 éthyle, (alcoxy (en $C_1$ à $C_4$)-carbonyl)-1 indolyl)-2 éthyle, (alcoxy (en $C_1$ à $C_4$)-carbonyl)-1 phényl-2 éthyle;

$R^2$ et $R^3$ représentent chacun, indépendamment l'un de l'autre, un atome d'hydrogène, un groupe alkyle (en $C_1$ à $C_5$) alcanoyle (en $C_1$ à $C_5$), alcanoyle (en $C_2$ à $C_5$) ; (substitué une, deux ou trois fois par un groupe amino, alcoxy (en $C_1$ à $C_4$) hydroxy alcanoyloxy (en $C_1$ à $C_4$), phényle ou halogéno), un groupe benzoyle, un groupe benzoyle (substitué une ou plusieurs fois par un atome d'halogène, un groupe alcoxy (en $C_1$ à $C_4$) alkyle (en $C_1$ à $C_4$), amino, $R^4(R^5)$N-, hydroxy, alcanoyloxy (en $C_1$ à $C_4$), mercapto, alkylmercapto (en $C_1$ à $C_4$), nitro, cyano, alcoxy (en $C_1$ à $C_4$)- carbonyle,-amidosulfonyle), un groupe pyridyl-carbonyle, cycloalkyl-carbonyle ayant 5 à 7 atomes de carbone dans le reste cycloalkyle, un groupe $-CH_2$ pouvant être également remplacé par -O-, -S- ou -N($R^{10}$)- ; un groupe (chloro-4 phénoxy)-acétyle ; ou bien $R^2$ et $R^3$ forment ensemble un groupe phénylène dicarbonyle vicinal, phénylène-sulfonyl-1 carbonyl-2, alkylène (en $C_2$ à $C_8$)-dicarbonyle, alcénylène (en $C_4$ à $C_8$)-dicarbonyle, la chaîne carbonée du groupe alkylène- ou alcénylène-dicarbonyle pouvant également être interrompue une ou plusieurs fois par -O-, ou -S-, -N($R^4$)- ou pouvant contenir un atome de type spiro, par l'intermédiaire duquel peut être fixé un noyau en position 4, 5, 6 ou 7 ; un groupe phénylène-sulfényl-1 carbonyle-2, phénylène-sulfényl-1 carbonyle-;

$R^4$ et $R^5$ représentent chacun un atome d'hydrogène ou un groupe alkyle (en $C_1$ à $C_4$);

$R^6$ représente un groupe alkylène (en $C_1$ à $C_4$)

$R^7$ et $R^8$ représentent, indépendamment l'un de l'autre, un atome d'hydrogène, un groupe alkyle (en $C_1$ à $C_4$), ou, avec l'atome d'azote auquel ils sont reliés, ils forment un noyau hétérocyclique saturé pentagonal ou hexagonal, qui peut aussi contenir -O- ou

-N- ;
|
R9

$R^9$ représente un atome d'hydrogène, un groupe alkyle (en $C_1$ à $C_4$), alcoxy (en $C_1$ à $C_4$), phényle, alkyl (en $C_1$ à $C_4$)-phényle, alcoxy (en $C_1$ à $C_4$)-phényle ;

$R^{10}$ représente un atome d'hydrogène, un groupe alcanoyle (en $C_1$ à $C_5$);

n vaut 1, 2 ou 3).

ou un ses sels d'addition d'acide pharmacologiquement acceptables de ce composé, en faisant réagir une diacétone-1,4 de formule II

$(R^2)(R^3)N\text{-}(CH_2)_n\text{-}CO\text{-}CH_2CH_2\text{-}CO\text{-}R$ (II)

ou un dicétal-1,4 de formule IIa

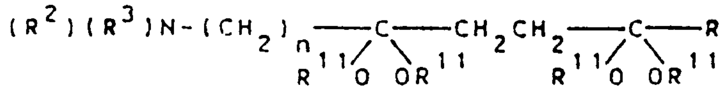

(IIa)

(dans laquelle $R^{11}$ représente un groupe alkyle ou deux radicaux $R^{11}$ voisins forment ensemble un groupe alkylène) avec une amine de formule

$H_2NR^1$ (III)

dans un solvant ou mélange de solvants inerte à des températures de 0 à 200°C et éventuellement on transforme en un sel d'addition d'acide et en ce qu'on isole le composé de formule I ou son sel d'addition d'acide, et l'on transforme la substance active ainsi obtenue, de façon connue en soi, par mélange avec un ou plusieurs excipients pharmacologiquement acceptables,en une préparation pharmaceutique administrable.